# EUROPÄISCHE PATENTSCHRIFT

(11) **EP 0 821 049 B1**
(45) Veröffentlichungstag und Bekanntmachung des Hinweises auf die Patenterteilung: **10.01.2001**
(21) Anmeldenummer: 97111985.4
(22) Anmeldetag: 15.07.1997
(51) Int. Cl.: C09K 19/30

(54) **Cyclopentyl-Verbindungen**
Cyclopentyl compounds
Composés du cyclopentyle

(30) Priorität: 26.07.1996 EP 96112064
(43) Veröffentlichungstag der Anmeldung: 28.01.1998
(73) Patentinhaber: Rolic AG, 6301 Zug (CH)
(72) Erfinder: Fünfschilling, Jürg, 4054 Basel (CH); Villiger, Alois, 4056 Basel (CH)
(74) Vertreter: Liebetanz, Michael, Dipl.-Phys.

(56) Entgegenhaltungen:
- WO-A-91/03446
- GB-A- 2 220 658
- DAVID M. COLLARD: "Control of thermal phase behavior of disklike molecules by modification of side-chain structure." JOURNAL OF THE AMERICAN CHEMICAL SOCIETY., Bd. 111, Nr. 5, 1989, DC US, Seiten 1829-1830, XP002043368

## Beschreibung

Die vorliegende Erfindung betrifft neue Cyclopentyl-Verbindungen, flüssigkristalline Mischungen, welche solche Verbindungen enthalten und die Verwendung solcher Verbindungen und Mischungen für optische und elektro-optische Vorrichtungen.

Flüssige Kristalle werden vor allem als Dielektrika in elektro-optischen Anzeige-Vorrichtungen verwendet, da die optischen Eigenschaften solcher Substanzen durch eine angelegte Spannung beeinflusst werden können. Solche elektro-optischen Vorrichtungen können auf verschiedenen physikalischen Effekten beruhen. Bekannt sind beispielsweise Zellen mit dynamischer Streuung, DAP-Zellen (Deformation aufgerichteter Phasen), Gast/Wirt-Zellen, TN-Zellen ("twisted nematic"), STN-Zellen ("super twisted nematic"), SBE-Zellen ("super birefrigrance effect"), OMI-Zellen ("optical mode interference") und aktiv adressierte AM-LCD's ("active matrix liquid crystal displays", Dünnfilmtransistor adressierte Zellen).

Neben den vorgenannten Zelltypen, deren Eigenschaften auf der Verwendung von nematischen oder cholesterischen Flüssigkristallen beruhen, sind auch Anzeigevorrichtungen bekannt, welche auf dem Prinzip von ferroelektrischen geneigt (tilted) chiral-smektischen Phasen beruhen. Als geneigt chiral-smektische Phasen eignen sich beispielsweise S_{C}*-, S_{F}*- und S_{F}*--Phasen, von denen die S_{C}*-Phase, wegen ihrer geringen Viskosität die kürzesten Ansprechzeiten ermöglicht. Bekannte Zelltypen, die auf dem Prinzip der S_{C}*-Phasen beruhen, sind beispielsweise SSF-Zellen ("surface stabilized ferroelectric"), SBF-Zellen ("short-pitch bistable ferroelectric") oder DHF-Zellen ("deformed helix ferroelectric").

Die Flüssigkristallmischungen müssen eine gute chemische und thermische Beständigkeit sowie eine hohe Stabilität gegenüber elektrischen und magnetischen Feldern besitzen. Ferner sollten sie geeignete Mesophasen über einen breiten Temperaturbereich, eine niedrige Viskosität und kurze Ansprechzeiten besitzen. Materialien auf der Basis von geneigten chiral-smektischen Phasen sollten ausserdem eine ausreichend hohe spontane Polarisation und je nach Zelltyp ein eher kleines (für SSF-Zellen) oder ein möglichst hohes (für SBF- und DHF-Zellen) Verdrillungsvermögen aufweisen. Um die Orientierung in der Zelle zu erleichtern, können sie ferner vorzugsweise eine S_{A}- oberhalb der S_{C}*-Phase aufweisen. Für DHF-Zellen ist für das Auftreten störungsfreier Bilder ein grosser Schaltwinkel zwischen den beiden stabilen Zuständen von grossem Vorteil.

Als ferroelektrische Flüssigkristallmischungen eignen sich vorzugsweise Gemische aus mindestens einem optisch aktiven Dotierstoff und einem Flüssigkristallmaterial, das aus mehreren achiralen Komponenten besteht, die in der Regel eine breite geneigt-smektische, vorzugsweise eine S_{C}-Phase aufweisen. Die optisch aktiven Dotierstoffe müssen selbst nicht flüssigkristallin sein, weisen aber vorzugsweise smektische oder cholesterische Phase(n) auf. Die Konzentration der optisch aktiven Dotierstoffe wird so gewählt, dass eine chirale geneigt-smektische (d.h. in der Regel eine S_{C}*-Phase) mit geeigneter Verdrillung sowie ausreichend hohe spontaner Polarisation induziert wird.

Gegenstand dieser Erfindung sind Cyclopentyl-Verbindungen der allgemeinen Formel 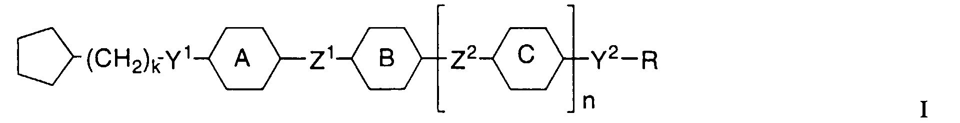 worin
- k: eine ganze Zahl von 4 bis 18;
- n: 0 oder 1;
- Y¹,Y²: eine Einfachbindung, -O-, -COO- oder -OOC-;
- die Ringe A, B, C: unabhängig voneinander gegebenenfalls mono- oder difluoriertes 1,4-Phenylen, Pyrimidin-2,5-diyl, Pyridin-2,5-diyl, Pyrazin-2,5-diyl, Naphthalin-2,6-diyl, Chinolin-2,6-diyl, Thiophen-2,5-diyl, Thiazol-2,5-diyl oder 1,3,4-Thiadiazol-2,5-diyl, und Ring C auch trans-1,4-Cyclohexylen oder trans-1,3-Dioxan-2,5-diyl;
- Z¹: eine Einfachbindung, -COO-, -OOC- oder -C≡C-;
- Z²: eine Einfachbindung, -COO-, -OOC-, -OCH₂-, -CH₂O- oder -(CH₂)₂-; und
- R: geradkettiges oder verzweigtes, gegebenenfalls optisch aktives, Alkyl oder Alkenyl mit 4 bis 20 Kohlenstoffatomen, worin eine oder zwei nicht benachbarte Methylen-Gruppen durch -O-, -COO-, -OOC- und/oder Epoxyethylen und/oder mindestens ein Wasserstoff durch Fluor und/oder ein Wasserstoff durch Chlor oder Cyan und/oder ein endständiger Wasserstoff durch Cyclopentyl ersetzt sein können, bedeuten.

Es wurde gefunden, dass die erfindungsgemässen Cyclopentyl-Verbindungen der allgemeinen Formel I im Vergleich zu konventionellen Verbindungen, die anstelle des endständigen Cyclopentan-Rings eine Ethyl- oder Vinyl-Gruppe tragen, leicht höhere oder vergleichbare Klärpunkte und Obergrenzen der S_{C}-Phase aufweisen, überraschenderweise aber den Schaltwinkel deutlich vergrössern, ohne die Schaltzeiten wesentlich zu beeinträchtigen. Die Verbindungen der Formel I besitzen zudem eine hohe chemische Beständigkeit sowie eine hohe Stabilität gegenüber elektrischen und magnetischen Feldern. Sie sind farblos, auf einfache Weise herstellbar und untereinander und in bekannten Flüssigkristallmaterialien gut löslich. Diese Eigenschaften bewirken, dass die Verwendung solcher Verbindungen in ferroelektrischen DHF-, SBF- oder SSF-Zellen störungsfreie Bilder mit sehr hohem Kontrast und kurzen Ansprechzeiten ermöglicht.

Der Ausdruck "geradkettiges oder verzweigtes, gegebenenfalls optisch aktives, Alkyl oder Alkenyl mit 4 bis 20 Kohlenstoffatomen, worin eine oder zwei nicht benachbarte Methylen-Gruppen durch -O-, -COO-, -OOC- und/oder Epoxyethylen und/oder mindestens ein Wasserstoff durch Fluor und/oder ein Wasserstoff durch Chlor oder Cyan und/oder ein endständiger Wasserstoff durch Cyclopentyl ersetzt sein können", umfasst im Rahmen der vorliegenden Erfindung Reste wie Alkyl, Alkenyl, Alkoxyalkyl, Alkenyloxyalkyl, Alkanoyloxyalkyl, Alkoxycarbonyl-alkyl, Fluoralkyl, Chloralkyl, Cyanalkyl, Trifluormethyl-alkyl, ω-Cyclopentyl-alkyl mit 4 bis 20 Kohlenstoffatomen.

Bevorzugt werden Verbindungen der Formel I, worin die Ringe A, B, und C unabhängig voneinander 1,4-Phenylen, 2- bzw. 3-Fluor-1,4-phenylen, 2,3-Difluor-1,4-phenylen, Pyrimidin-2,5-diyl, Pyridin-2,5-diyl oder Pyrazin-2,5-diyl und Ring C auch trans-1,4-Cyclohexylen oder trans-1,3-Dioxan-2,5-diyl bedeutet. Ganz besonders bevorzugt werden Verbindungen der Formel I, worin mindestens einer der Ringe A, B oder C 1,4-Phenylen, 2- bzw. 3-Fluor-1,4-phenylen oder 2,3-Difluor-1,4-phenylen bedeutet und höchstens einer der Ringe A, B oder C Pyrimidin-2,5-diyl, Pyridin-2,5-diyl oder Pyrazin-2,5-diyl bedeutet.

Besonders bevorzugt sind die Verbindungen der Formeln 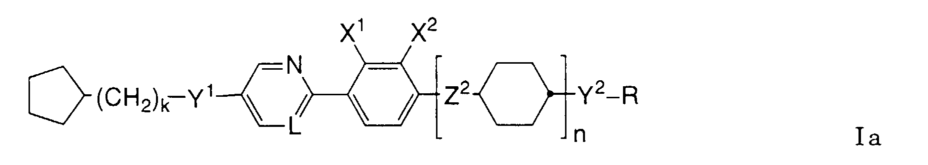 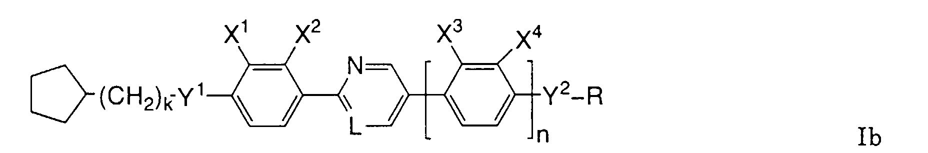 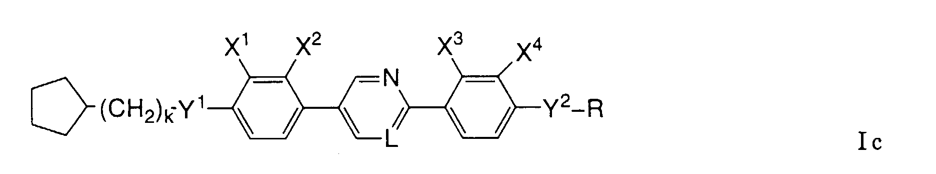 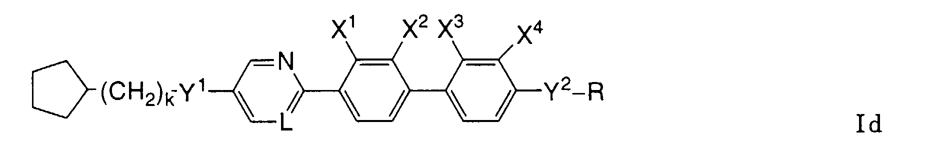 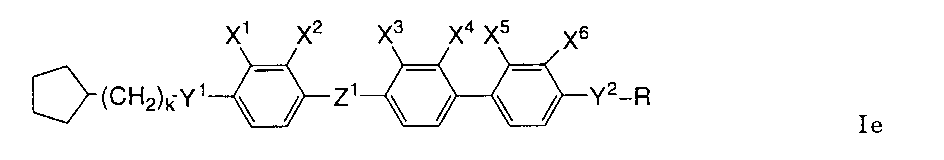 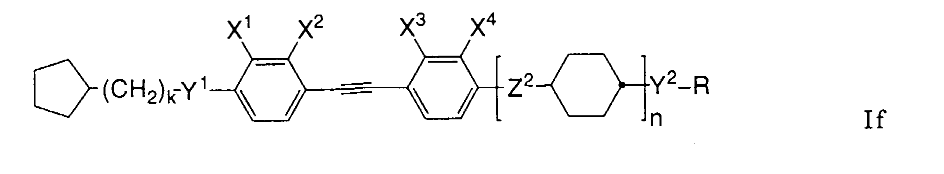 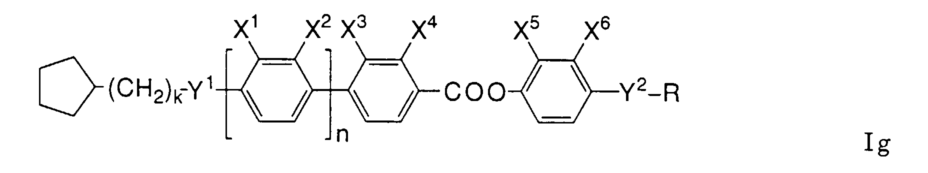 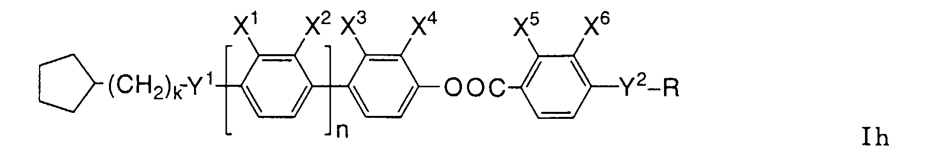 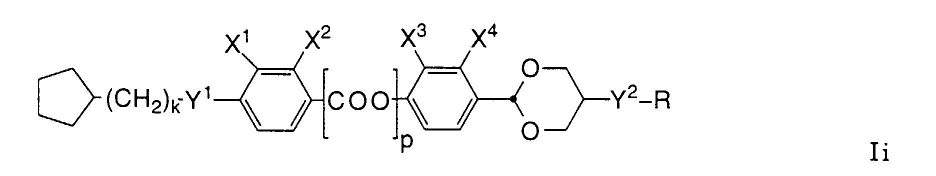 worin
- L: Stickstoff oder -CH=;
- X¹⁻⁶: Wasserstoff oder Fluor;
- p: 0 oder 1 bedeuten; und
- n, k, Y¹, Y² und R: die oben angegebenen Bedeutungen haben.

Weiter werden Verbindungen der Formel I und Ia-Ii bevorzugt, worin k eine ganze Zahl von 5 bis 12 bedeutet. Vorzugsweise bedeutet Y¹ eine Einfachbindung, -O- oder -COO-; insbesondere werden Verbindungen der Formel I und Ia-Ii bevorzugt, worin Y¹ -O- bedeutet.

Die Verbindungen der Formel I können, je nach Beschaffenheit des Restes R, als optisch aktive Dotierstoffe oder als achirale Mischungskomponenten für Flussigkristallmischungen eingesetzt werden.

Besonders geeignete optisch aktive Verbindungen der Formel I und Ia-Ii sind diejenigen Verbindungen, worin der Rest R ein Alkyl- oder Alkenyl-Rest mit 5 bis 12 Kohlenstoffatomen, worin 1 bis 2 nicht endständige Methylengruppen durch -C*H(W)-, -C*F(CH₃)- und/oder Epoxyethylen ersetzt sind und 1 oder 2 nicht benachbarte Methylengruppen durch -O-, -COO-, -OOC- ersetzt sein können. W bedeutet Fluor, Chlor, Cyan, Methyl oder Trifluormethyl und C* ein chirales Zentrum.

In besonders bevorzugten optisch aktiven Verbindungen der Formel I und Ia-Ii bedeutet die Gruppe -Y²-R eine optisch aktive Gruppe wie 2- oder 3-Fluoralkyl, 2- oder 3-Fluoralkoxy, 2,3-Difluoralkoxy, 2- oder 3-Fluor-alkanoyloxy, 2,3-Difluor-alkanoyloxy, 2-Fluor-2-methyl-alkanoyloxy, 2-Fluor-3-methyl-alkanoyloxy, 2- oder 3-Chlor-alkoxy, 2- oder 3-Chlor-alkanoyloxy, 2-Chlor-3-methyl-alkanoyloxy, 1- oder 2-Cyanalkyl, 1- oder 2-Cyanalkoxy, 2-oder 3-Cyan-alkanoyloxy, 1-, 2- oder 3-Methylalkyl, 1-, 2- oder 3-Methylalkoxy, 2- oder 3-Methyl-alkanoyloxy, 1-, 2- oder 3-Trifluormethyl-alkanoyloxy, 1,2-Epoxyalkyl, 2,3-Epoxyalkoxy, 2,3-Epoxy-alkanoyloxy, 1-Alkoxycarbonyl-ethyl, 1-Alkoxycarbonyl-ethoxy, 2-Alkoxy-propanoyloxy, (1-Methyl-alkoxy)-carbonyl, (1-Trifluormethyl-alkoxy)-carbonyl, 1-Alkoxy-2,2,2-trifluorethyl, ω-Trifluormethyl-ω-alkoxyalkyl mit jeweils 5 bis 12 Kohlenstoffatomen bedeutet.

Als achirale Mischungskomponenten für Flüssigkristallmischungen eignen sich insbesondere Verbindungen der allgemeinen Formel I und Ia-Ii, worin Y² eine Einfachbindung, -O- oder -OOC- und R ein geradkettiger oder verzweigter (racemischer) Alkyl- oder Alkenyl-Rest mit 5 bis 12 Kohlenstoffatomen bedeutet, worin eine nicht mit Y2 benachbarte Methylen-Gruppe durch -O-, -COO- oder -OOC- und/oder ein oder mehrere Wasserstoffatome durch Fluor und/oder ein endständiges Wasserstoffatom durch Cyclopentyl ersetzt sein kann, solche bevorzugten Reste sind beispielsweise 4-, 5-, 6-, 7- oder 8-Methyl-alkyl, 1-, 2-, 3-, 4-, 5-, 6- oder 7-Alkenyl, ω-Alkoxy-alkyl, ω-Alkoxycarbonyl-alkyl, ω-Alkanoyloxy-alkyl, ω-Perfluoralkyl-alkyl, ω-Cyclopentyl-alkyl und dergleichen.

Besonders bevorzugte optisch inaktive Reste R sind geradkettige oder methyl-verzweigte Alkyl- oder Alkenyl-Reste mit 5 bis 12 Kohlenstoffatomen wie beipielsweise Pentyl, Hexyl, Heptyl, Octyl, Nonyl, Decyl, Undecyl, Dodecyl, 1-, 2-, 3- oder 4-Pentenyl, 1-, 2-, 3- oder 4-Hexenyl, 1-, 2-, 3-, 4- oder 5-Heptenyl, 1-, 2-, 3-, 4-, 5- oder 6-Octenyl, 1-, 2-, 3-, 4-, 5-, 6- oder 7-Nonenyl, 1-, 2-, 3-, 4-, 5-, 6- oder 7-Decenyl, 1-, 2-, 3-, 4-, 5-, 6- oder 7-Undecenyl, 1-, 2-, 3-, 4-, 5-, 6- oder 7- Dodecenyl, 4-Methyl-hexyl, 4- oder 5-Methyl-heptyl, 4-, 5- oder 6-Methyl-octyl, 4-, 5-, 6- oder 7-Methyl-nonyl, 4-, 5-, 6-, 7- oder 8-Methyl-decyl, 4-, 5-, 6-, 7-, 8- oder 9-Methyl-undecyl, 4-, 5-, 6-, 7-, 8- 9- oder 10-Methyl-Dodecyl und dergleichen.

Die erfindungsgemässen Verbindungen der Formel I können in an sich bekannter Weise, analog wie Verbindungen, die anstelle des endständigen Cyclopentan-Rings ein Wasserstoffatom tragen, hergestellt werden. Die Reaktionsführung hängt dabei weitgehend von den funktionellen Gruppen in Y¹, Y², Z¹, Z² und R ab.

Verbindungen der Formel I, worin Y¹ eine Einfachbindung bedeutet, werden vorzugsweise über eine Wittig-Reaktion eines Zwischenproduktes der Formel 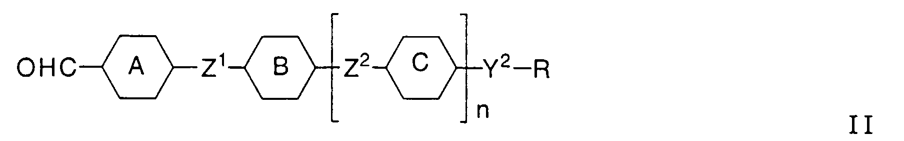 mit einem (ω-Cyclopentyl)-alkyl-triphenyl-phosphoniumhalogenid unter Zusatz eines Aequivalents einer geeigneten Base, wie beispielsweise Kalium-tert.-butylat, in einem inerten Lösungsmittel wie beispielsweise tert. Butylmethylether, Diethylether oder Tetrahydrofuran, vorzugsweise zwischen -20 °C und Raumtemperatur und anschliessender katalytischer Hydrierung, z. Bsp. mit Palladium auf Kohle in Toluol und dergleichen, hergestellt.

Verbindungen der Formel I, worin Y¹ -O- bedeutet, können durch Alkylierung eines Zwischenproduktes der Formel 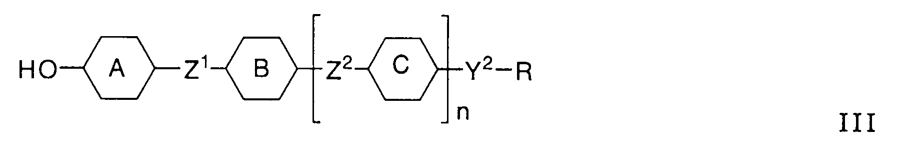 mit einem (ω-Brom-alkyl)-cyclopentan und einer geeigneten Base, wie zum Beispiel Kaliumcarbonat, in einem polaren Lösungsmittel wie beispielsweise DMF, Ethyl-methylketon oder DMSO, bei einer Temperatur zwischen Raumtemperatur und 100 °C, vorzugsweise bei etwa 50 °C, hergestellt werden.

Verbindungen der Formel I, worin Y¹ -COO- bedeutet, können durch Acylierung eines Zwischenproduktes der Formel III mit einer ω-Cyclopentylalkansäure beispielsweise in Gegenwart eines leichten Ueberschusses an N,N'-Dicyclohexyl-carbodiimid und einer katalytischen Menge 4-Dimethylamino-pyridin in Dichlormethan bei 0 °C oder Raumtemperatur hergestellt werden.

Die obgenannten Verfahren sind Standardreaktionen der organischen Chemie und dem Fachmann bekannt. Die verwendeten Zwischenprodukte sind bekannte oder Analoge bekannter Verbindungen.

Die Verbindungen der Formel **I** können in Form von Gemischen untereinander und/oder mit anderen Flüssigkristallkomponenten verwendet werden. Die Erfindung betrifft somit ebenfalls flüssigkristalline Mischungen mit mindestens 2 Komponenten, welche dadurch gekennzeichnet sind, dass mindestens 1 Komponente eine Verbindung der Formel **I** ist. Eine zweite und gegebenenfalls weitere Komponenten können weitere Verbindungen der Formel **I** oder andere geeignete Flüssigkristall-Komponenten sein. Die Verbindungen der Formel **I** werden vorzugsweise als Bestandteil von Mischungen, welche eine geneigte smektische Phase, beispielsweise eine S_{C}*-Phase, aufweisen, verwendet. Die Verbindungen der Formel **I** können dabei als optisch inaktive Basiskomponenten und/oder als optisch aktive Dotierstoffe eingesetzt werden.

Aufgrund der guten Löslichkeit der erfindungsgemässen Verbindungen der Formel **I** in anderen Flüssigkristallkomponenten und aufgrund ihrer guten Mischbarkeit untereinander kann der Anteil an Verbindungen der Formel **I** in den erfindungsgemässen Mischungen relativ hoch sein und beispielsweise 0,5-30 Gew.-% betragen. Werden optisch inaktive Verbindungen der Formel **I** eingesetzt, so ist im allgemeinen ein Anteil von etwa 2-30 Gew.-%, insbesondere von 3-25 Gew.-% bevorzugt. Der Anteil optisch aktiver Dotierstoffe wird weitgehend durch das Verdrillungsvermögen, die spontane Polarisation und die gewünschte Ganghöhe der Mischung bestimmt. Der Anteil gegebenenfalls eingesetzter optisch aktiver Dotierstoffe der Formel **I** kann daher in einem breiten Bereich variieren und beispielsweise 0,5-20 Gew.-%, insbesondere etwa 1-15 Gew.-%, betragen.

Vorzugsweise enthalten die erfindungsgemässen Mischungen neben einer oder mehreren Verbindungen der Formel **I** eine oder mehrere Verbindungen aus der Gruppe der Verbindungen der allgemeinen Formeln 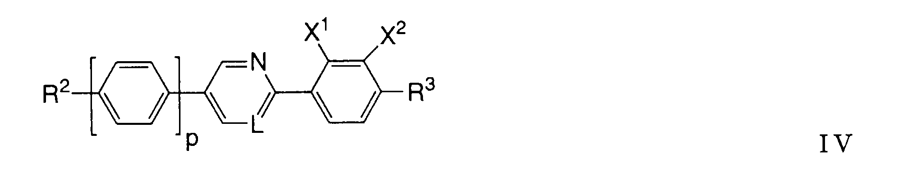 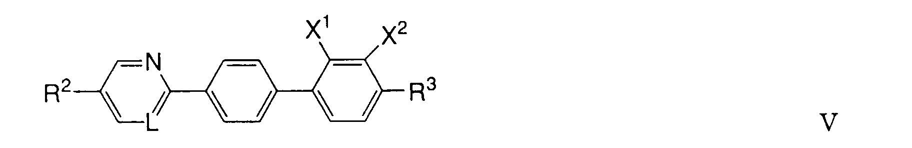 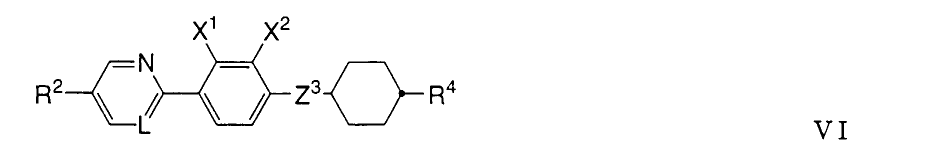 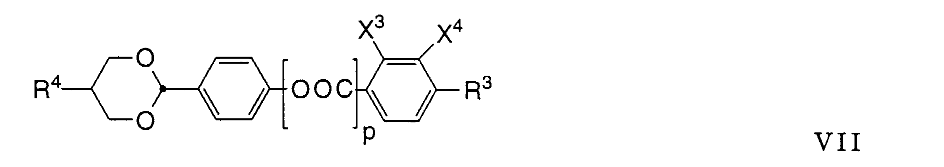 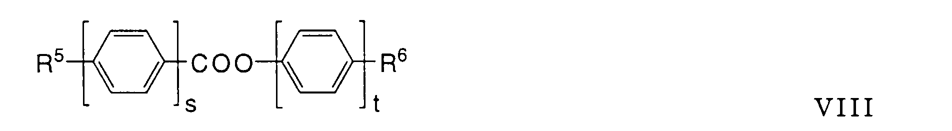 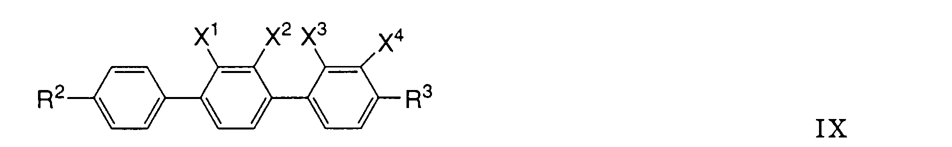 worin
- p: 0 oder 1;
- s, t: 1 oder 2 ist, wobei s + t = 2 oder 3;
- L: Stickstoff oder -CH=;
- X¹, X², X³, X⁴: unabhängig voneinander Wasserstoff oder Fluor;
- Z³: eine Einfachbindung, -OOC-, -OCH₂- oder -(CH₂)₂-;
- R², R³: unabhängig voneinander Alkyl, Alkenyl, Alkoxy, Alkoxyalkyl, Alkenyloxy, Alkanoyloxy, Alkenoyloxy, Alkoxyalkoxy, Alkoxycarbonyl oder Alkenyloxycarbonyl;
- R⁴: Alkyl oder Alkenyl;
- R⁵, R⁶: unabhängig voneinander Alkyl, Alkenyl, Alkoxy oder Alkenyloxy bedeuten.

Die Substituenten R² bis R⁶ können geradkettig oder verzweigt sein, sind jedoch bevorzugt geradkettig. Sie besitzen höchstens 18, bevorzugt jedoch 5 bis 12 Kohlenstoffatome.

Die erfindungsgemässen Mischungen können zusätzlich eine oder mehrere optisch aktive Verbindungen aus der Gruppe der Verbindungen der allgemeinen Formeln 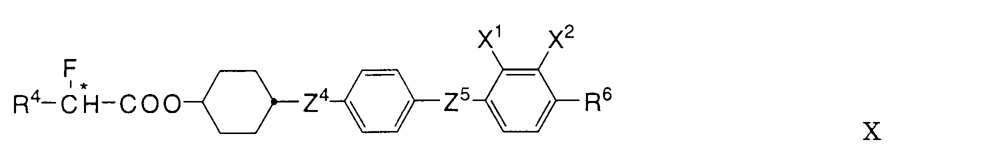 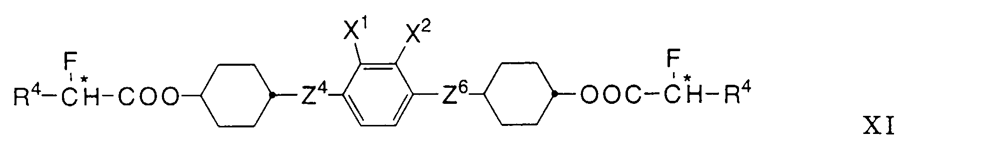 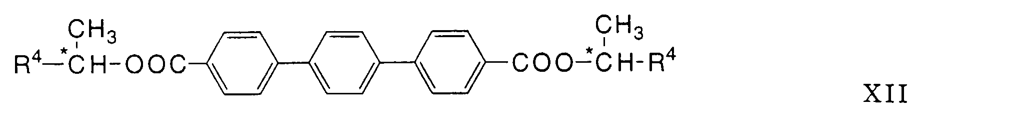 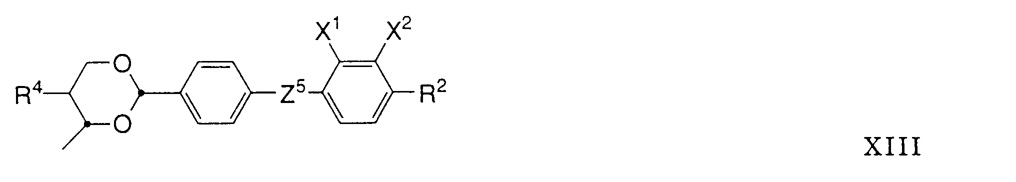 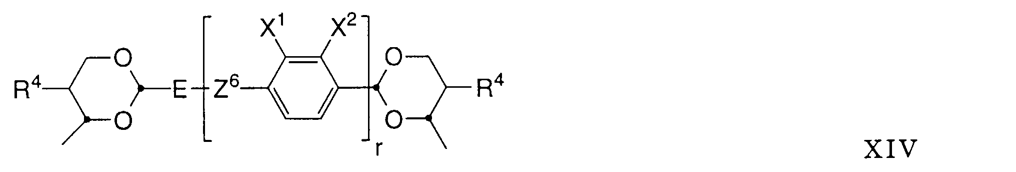 worin
- E: 1,4-Phenylen oder trans-1,4-Cyclohexylen;
- r: 0, 1 oder 2;
- Z⁴: eine Einfachbindung, -(CH₂)₂- oder -CH₂O-;
- Z⁵: eine Einfachbindung, -OCH₂-, -COO- oder -OOC-;
- Z⁶: eine Einfachbindung, -(CH₂)₂- oder -OCH₂- bedeuten;
und die übrigen Symbole die oben genannten Bedeutungen haben.

Die Herstellung der flüssigkristallinen Mischungen und der elektrooptischen Vorrichtung kann in an sich bekannter Weise erfolgen.

Die Herstellung der Verbindungen der Formel I und von flüssigkristallinen Mischungen mit solchen Verbindungen werden durch die folgenden Beispiele weiter veranschaulicht. Die zur Charakterisierung der Phasenumwandlungen verwendeten Abkürzungen haben folgende Bedeutungen: C steht für kristallin, S, S_{A}, S_{C} usw. stehen für smektisch, smektisch A, smektisch C usw., N steht für nematisch und I für isotrop.

### Beispiel 1

Ein Gemisch aus 0,157 g 4-(5-Heptyl-2-pyridyl)-phenol, 0,153 g (8-Bromoctyl)-cyclopentan, 6 ml Dimethylformamid und 0,24 g fein pulverisiertem Kaliumcarbonat wurde 3 Stunden bei 55 °C gerührt. Das Reaktionsgemisch wurde zwischen Wasser und Diethylether verteilt. Die organische Phase wurde mit Wasser und mit gesättigter Kochsalzlösung gewaschen, über Natriumsulfat getrocknet, filtriert und das Filtrat eingeengt. Chromatographie des Rohprodukts (0,28 g) an 10 g Kieselgel mit Hexan/Ethylacetat 37:3 (v/v) und zweimalige Umkristallisation der produkthaltigen Fraktionen aus je 3 ml Hexan ergab 0,16 g reines 5-Heptyl-2-[4-(8-cyclopentyl-octyloxy)-phenyl]-pyridin; Smp. (C-S_{I}) 38,3 °C, S_{I}-S_{C} 56,9 °C, Klp. (S_{C}-I) 72,6 °C.

Das als Ausgangsmaterial eingesetzte (8-Bromoctyl)-cyclopentan wurde wie folgt hergestellt:

Zu einer Lösung von 2,27 g Cyclopentan-octanol und 3,22 g Triphenylphosphin in 40 ml Dichlormethan wurden im Eisbad portionenweise 2,4 g N-Bromsuccinimid gegeben. Das Reaktionsgemisch wurde über Nacht bei Raumtemperatur stehen gelassen, filtriert und das Filtrat eingeengt. Der Rückstand wurde zwischen 50 ml Methanol/Wasser (vol.) 4:1 und 40 ml Hexan verteilt und die Methanol-Phase abgetrennt. Die Hexan-Phase wurde zweimal mit je 20 ml Methanol/Wasser 4:1 gewaschen und eingeengt. Destillation des Rückstands im Kugelrohr bei 130-150 °C / 0,05 Torr ergab 2,3 g (8-Bromoctyl)-cyclopentan als farbloses Oel.

Analog können folgende Verbindungen hergestellt werden:
5-Octyl-2-[4-(8-cyclopentyl-octyloxy)-phenyl]-pyridin;
5-Nonyl-2-[4-(8-cyclopentyl-octyloxy)-phenyl]-pyridin, Smp. (C-S_{I}) 53,2 °C, S_{I}-S_{C} 67,1 °C, Klp. (S_{C}-I) 77,0 °C;
5-Decyl-2-[4-(8-cyclopentyl-octyloxy)-phenyl]-pyridin;
5-Heptyl-2-[4-(7-cyclopentyl-heptyloxy)-phenyl]-pyridin, (C-S_{C}) 73,2 °C, S_{I}-S_{C} 70,0 °C, Klp. (S_{C}-I) 78,2 °C;
5-Octyl-2-[4-(7-cyclopentyl-heptyloxy)-phenyl]-pyridin;
5-Nonyl-2-[4-(7-cyclopentyl-heptyloxy)-phenyl]-pyridin, Smp. (C-S_{I}) 73,2 °C, S_{I}-S_{C} 78,9 °C, Klp. (S_{C}-I) 82,6 °C;
5-Decyl-2-[4-(7-cyclopentyl-heptyloxy)-phenyl]-pyridin;
5-Heptyl-2-[4-(6-cyclopentyl-hexyloxy)-phenyl]-pyridin;
5-Nonyl-2-[4-(6-cyclopentyl-hexyloxy)-phenyl]-pyridin;
5-Nonyl-2-[4-(5-cyclopentyl-pentyloxy)-phenyl]-pyridin;
5-Heptyl-2-[4-(9-cyclopentyl-nonyloxy)-phenyl]-pyrimidin;
5-Heptyl-2-[4-(8-cyclopentyl-octyloxy)-phenyl]-pyrimidin;
5-Octyl-2-[4-(8-cyclopentyl-octyloxy)-phenyl]-pyrimidin, Smp. (C-S_{C}) 40,2 °C, S_{C}-S_{A} 55,6 °C, S_{A}-N 57,6 °C, Klp. (N-I) 61,5 °C;
5-Nonyl-2-[4-(8-cyclopentyl-octyloxy)-phenyl]-pyrimidin, Smp. (C-S_{C}) 50,6 °C, S_{C}-S_{A} 63,6 °C, Klp. (S_{A}-I) 61,5 °C;
5-Decyl-2-[4-(8-cyclopentyl-octyloxy)-phenyl]-pyrimidin;
5-Heptyl-2-[4-(7-cyclopentyl-heptyloxy)-phenyl]-pyrimidin;
5-Octyl-2-[4-(7-cyclopentyl-heptyloxy)-phenyl]-pyrimidin, Smp. (C-S_{C}) 52,7 °C, S_{C}-N 57,5 °C, Klp. (N-I) 66,0 °C;
5-Nonyl-2-[4-(7-cyclopentyl-heptyloxy)-phenyl]-pyrimidin, Smp. (C-S_{C}) 57,5 °C, S_{C}-S_{A} 68,6 °C, S_{A}-N 70,4 °C, Klp. (N-I) 71,1 °C;
5-Decyl-2-[4-(7-cyclopentyl-heptyloxy)-phenyl]-pyrimidin;
5-Octyl-2-[4-(6-cyclopentyl-hexyloxy)-phenyl]-pyrimidin;
5-Nonyl-2-[4-(6-cyclopentyl-hexyloxy)-phenyl]-pyrimidin;
5-Nonyl-2-[4-(5-cyclopentyl-pentyloxy)-phenyl]-pyrimidin;
5-Heptyl-2-[4-(7-cyclopentyl-heptyloxy)-3-fluor-phenyl]-pyrimidin;
5-Octyl-2-[4-(7-cyclopentyl-heptyloxy)-3-fluor-phenyl]-pyrimidin;
5-Nonyl-2-[4-(7-cyclopentyl-heptyloxy)-3-fluor-phenyl]-pyrimidin;
5-Nonyl-2-[4-(7-cyclopentyl-heptyloxy)-2-fluor-phenyl]-pyrimidin;
5-Heptyl-2-[4-(7-cyclopentyl-heptyloxy)-2,3-difluor-phenyl]-pyrimidin;
5-Octyl-2-[4-(7-cyclopentyl-heptyloxy)-2,3-difluor-phenyl]-pyrimidin;
5-Nonyl-2-[4-(7-cyclopentyl-heptyloxy)-2,3-difluor-phenyl]-pyrimidin;
5-Decyl-2-[4-(7-cyclopentyl-heptyloxy)-2,3-difluor-phenyl]-pyrimidin;
5-(7-Cyclopentyl-heptyloxy)-2-(4-octyl-phenyl)-pyrimidin;
5-(7-Cyclopentyl-heptyloxy)-2-(4-nonyl-phenyl)-pyrimidin;
5-(7-Cyclopentyl-heptyloxy)-2-(4-decyl-phenyl)-pyrimidin;
5-(8-Cyclopentyl-octyloxy)-2-(4-heptyl-phenyl)-pyrimidin;
5-(8-Cyclopentyl-octyloxy)-2-(4-octyl-phenyl)-pyrimidin;
5-(8-Cyclopentyl-octyloxy)-2-(4-nonyl-phenyl)-pyrimidin;
5-(8-Cyclopentyl-octyloxy)-2-(4-decyl-phenyl)-pyrimidin;
5-(9-Cyclopentyl-nonyloxy)-2-(4-octyl-phenyl)-pyrimidin;
5-(9-Cyclopentyl-nonyloxy)-2-(4-nonyl-phenyl)-pyrimidin;
5-Hexyloxy-2-[4-(8-cyclopentyl-octyloxy)-phenyl]-pyrimidin;
5-Heptyloxy-2-[4-(8-cyclopentyl-octyloxy)-phenyl]-pyrimidin;
5-Octyloxy-2-[4-(8-cyclopentyl-octyloxy)-phenyl]-pyrimidin;
5-Nonyloxy-2-[4-(8-cyclopentyl-octyloxy)-phenyl]-pyrimidin, Smp. (C-S_{C}) 66,5 °C, S_{C}-S_{A} 89,4 °C, Klp. (S_{A}-I) 91,5 °C;
5-Decyloxy-2-[4-(8-cyclopentyl-octyloxy)-phenyl]-pyrimidin, Smp. (C-S_{C}) 64,1 °C, Klp. (S_{C}-I) 94,5 °C;
5-Heptyloxy-2-[4-(7-cyclopentyl-heptyloxy)-phenyl]-pyrimidin;
5-Octyloxy-2-[4-(7-cyclopentyl-heptyloxy)-phenyl]-pyrimidin, Smp. (C-S_{C}) 65,5 °C, S_{C}-S_{A} 88,1 °C, S_{A}-N 93,3 °C, Klp. (N-I) 94,4 °C;
5-Nonyloxy-2-[4-(7-cyclopentyl-heptyloxy)-phenyl]-pyrimidin, Smp. (C-S_{C}) 65,7 °C, S_{C}-S_{A} 93,2 °C, Klp. (S_{A}-I) 94,6 °C;
5-Decyloxy-2-[4-(7-cyclopentyl-heptyloxy)-phenyl]-pyrimidin, Smp. (C-S_{C}) 65,1 °C, Klp. (S_{C}-I) 97,7 °C;
5-Oct-2E-enyloxy-2-[4-(7-cyclopentyl-heptyloxy)-phenyl]-pyrimidin;
5-Oct-4E-enyloxy-2-[4-(7-cyclopentyl-heptyloxy)-phenyl]-pyrimidin;
5-Oct-5Z-enyloxy-2-[4-(7-cyclopentyl-heptyloxy)-phenyl]-pyrimidin;
5-(4-Methyl-octyloxy-2-[4-(7-cyclopentyl-heptyloxy)-phenyl]-pyrimidin;
5-(5-Methyl-octyloxy-2-[4-(7-cyclopentyl-heptyloxy)-phenyl]-pyrimidin;
5-(6-Methyl-octyloxy-2-[4-(7-cyclopentyl-heptyloxy)-phenyl]-pyrimidin;
5-(7-Cyclopentyl-heptyloxy)-2-[4-(7-cyclopentyl-heptyloxy)-phenyl]-pyrimidin;
5-(8-Cyclopentyl-octyloxy)-2-[4-(8-cyclopentyl-octyloxy)-phenyl]-pyrimidin; Smp. (C-S_{C}) 87,4 °C, Klp. (S_{C}-I) 88,4 °C;
5-(7-Cyclopentyl-heptyloxy)-2-(4-heptyloxy-phenyl)-pyrimidin;
5-(7-Cyclopentyl-heptyloxy)-2-(4-octyloxy-phenyl)-pyrimidin, Smp. (C-S_{C}) 65,2 °C, Klp. (S_{C}-I) 98,2 °C;
5-(7-Cyclopentyl-heptyloxy)-2-(4-nonyloxy-phenyl)-pyrimidin;
5-(7-Cyclopentyl-heptyloxy)-2-(4-decyloxy-phenyl)-pyrimidin;
5-(8-Cyclopentyl-octyloxy)-2-(4-hexyloxy-phenyl)-pyrimidin;
5-(8-Cyclopentyl-octyloxy)-2-(4-heptyloxy-phenyl)-pyrimidin;
5-(8-Cyclopentyl-octyloxy)-2-(4-octyloxy-phenyl)-pyrimidin;
5-(8-Cyclopentyl-octyloxy)-2-(4-nonyloxy-phenyl)-pyrimidin;
(R)-5-(6-Cyclopentyl-hexyloxy)-2-[4-(2-fluor-octyloxy)-phenyl]-pyrimidin;
(R)-5-(7-Cyclopentyl-heptyloxy)-2-[4-(2-fluor-octyloxy)-phenyl]-pyrimidin;
(R)-5-(8-Cyclopentyl-octyloxy)-2-[4-(2-fluor-octyloxy)-phenyl]-pyrimidin;
(R)-5-(9-Cyclopentyl-nonyloxy)-2-[4-(2-fluor-octyloxy)-phenyl]-pyrimidin;
(R)-5-(10-Cyclopentyl-decyloxy)-2-[4-(2-fluor-octyloxy)-phenyl]-pyrimidin;
(S)-5-(7-Cyclopentyl-heptyloxy)-2-[4-(3-fluor-octyloxy)-phenyl]-pyrimidin;
(S,S)-5-(7-Cyclopentyl-heptyloxy)-2-[4-(2,3-difluor-octyloxy)-phenyl]-pyrimidin;
(S,S)-5-(7-Cyclopentyl-heptyloxy)-2-[4-(2,3-epoxy-nonyloxy)-phenyl]pyrimidin;
(S,S)-5-(8-Cyclopentyl-octyloxy)-2-[4-(2,3-epoxy-nonyloxy)-phenyl]pyrimidin;
(R)-5-(7-Cyclopentyl-heptyloxy)-2-[4-(2-chlor-octyloxy)-phenyl]-pyrimidin;
(S)-5-(7-Cyclopentyl-heptyloxy)-2-[4-(3-chlor-octyloxy)-phenyl]-pyrimidin;
5-Octyl-2-[4-(7-cyclopentyl-heptyloxy)-2-fluor-phenyl]-pyrimidin;
5-Nonyl-2-[4-(7-cyclopentyl-heptyloxy)-2-fluor-phenyl]-pyrimidin;
5-Heptyl-2-[4-(7-cyclopentyl-heptyloxy)-3-fluor-phenyl]-pyrimidin;
5-Octyl-2-[4-(7-cyclopentyl-heptyloxy)-3-fluor-phenyl]-pyrimidin;
5-Nonyl-2-[4-(7-cyclopentyl-heptyloxy)-3-fluor-phenyl]-pyrimidin;
5-Heptyl-2-[4-(7-cyclopentyl-heptyloxy)-2,3-difluor-phenyl]-pyrimidin;
5-Octyl-2-[4-(7-cyclopentyl-heptyloxy)-2,3-difluor-phenyl]-pyrimidin;
5-Nonyl-2-[4-(7-cyclopentyl-heptyloxy)-2,3-difluor-phenyl]-pyrimidin;
5-Decyl-2-[4-(7-cyclopentyl-heptyloxy)-2,3-difluor-phenyl]-pyrimidin;
5-Heptyloxy-2-[4-(7-cyclopentyl-heptyloxy)-2-fluor-phenyl]-pyrimidin;
5-Heptyloxy-2-[4-(7-cyclopentyl-heptyloxy)-2,3-difluor-phenyl]-pyrimidin;
5-Octyloxy-2-[4-(7-cyclopentyl-heptyloxy)-2,3-difluor-phenyl]-pyrimidin;
5-Nonyloxy-2-[4-(7-cyclopentyl-heptyloxy)-2,3-difluor-phenyl]-pyrimidin;
5-(7-Cyclopentyl-heptyloxy)-2-(2,3-difluor-4-octyl-phenyl)-pyrimidin;
5-(7-Cyclopentyl-heptyloxy)-2-(2,3-difluor-4-nonyl-phenyl)-pyrimidin;
(R)-5-(7-Cyclopentyl-heptyloxy)-2-[2-fluor-4-(2-fluor-octyloxy)-phenyl]pyrimidin;
(R)-5-(7-Cyclopentyl-heptyloxy)-2-[3-fluor-4-(2-fluor-octyloxy)-phenyl]pyrimidin;
(R)-5-(5-Cyclopentyl-pentyloxy)-2-[2,3-difluor-4-(2-fluor-octyloxy)-phenyl]pyrimidin;
(R)-5-(6-Cyclopentyl-hexyloxy)-2-[2,3-difluor-4-(2-fluor-octyloxy)-phenyl]pyrimidin;
(R)-5-(7-Cyclopentyl-heptyloxy)-2-[2,3-difluor-4-(2-fluor-octyloxy)-phenyl]pyrimidin;
(R)-5-(7-Cyclopentyl-heptyloxy)-2-[2,3-difluor-4-(2-fluor-octyloxy)-phenyl]pyrimidin;
4-[5-(7-Cyclopentyl-heptyloxy)-pyrimidin-2-yl]-phenyl-(R)-2-fluor-octanoat;
4-[5-(7-Cyclopentyl-heptyloxy)-pyrimidin-2-yl]-phenyl-(R)-2-chloroctanoat;
4-[5-(7-Cyclopentyl-heptyloxy)-pyrimidin-2-yl]-phenyl-(S)-2-cyan-octanoat;
4-[5-(7-Cyclopentyl-heptyloxy)-pyrimidin-2-yl]-phenyl-(S)-2-methyl-octanoat;
4- [5-(7-Cyclopentyl-heptyloxy)-pyrimidin-2-yl] -phenyl-(R)-2-(trifluormethyl)-octanoat;
4-[5-(7-Cyclopentyl-heptyloxy)-pyrimidin-2-yl]-phenyl-(R)-2-fluor-2-methyl-octanoat;
4-[5-(8-Cyclopentyl-octyloxy)-pyrimidin-2-yl]-phenyl-(2R,3S)-2,3-epoxy-octanoat;
5-Nonyloxy-2-[4-(8-cyclopentyl-octyloxy)-phenyl]-pyrazin;
4-Nonyloxy-4'-(8-cyclopentyl-octyloxy)-tolan;
4,4'-Bis(7-cyclopentyl-heptyloxy)-tolan;
4,4'-Bis(8-cyclopentyl-octyloxy)-tolan;
4-Nonyl-benzoesäure-4-(8-cyclopentyl-octyloxy)-phenylester;
4-Decyloxy-benzoesäure-4-(8-cyclopentyl-octyloxy)-phenylester;
4-Decyloxy-benzoesäure-3-fluor-4-(8-cyclopentyl-octyloxy)-phenylester;
4"-Heptyl-4-(4-cyclopentyl-butyloxy)-2'-fluor-[1,1';4'-1"]terphenyl;
4"-Heptyl-4-(4-cyclopentyl-butyloxy)-3-fluor-[1,1';4'-1']terphenyl;
4"-Hexyl-4-(4-cyclopentyl-butyloxy)-2",3"-difluor-[1,1';4'-1"]terphenyl;
4"-Heptyl-4-(4-cyclopentyl-butyloxy)-2,3-difluor-[1,1';4'-1"]terphenyl;
4"-Heptyl-4-(5-cyclopentyl-pentyloxy)-2'-fluor-[1,1';4'-1"]terphenyl;
4"-Heptyl-4-(5-cyclopentyl-pentyloxy)-3-fluor-[1,1';4'-1"]terphenyl;
4"-Hexyl-4-(5-cyclopentyl-pentyloxy)-2",3"-difluor-[1,1';4'-1"]terphenyl;
4"-Heptyl-4-(5-cyclopentyl-pentyloxy)-2,3-difluor-[1,1';4'-1"]terphenyl;
4"-Hexyl-4-(5-cyclopentyl-pentyloxy)-3,2"-difluor-[1,1';4'-1"]terphenyl;
trans-5-Octyl-2-[4'-(5-cyclopentyl-pentyloxy)-biphenyl-4-yl]-1,3-dioxan;
trans-5-Nonyl-2-[4'-(5-cyclopentyl-pentyloxy)-biphenyl-4-yl]-1,3-dioxan;
trans-5-Octyl-2-[4'-(5-cyclopentyl-pentyloxy)-3'-fluor-biphenyl-4-yl]-1,3-dioxan;
trans-5-Nonyl-2-[4'-(5-cyclopentyl-pentyloxy)-3'-fluor-biphenyl-4-yl]-1,3-dioxan;
4-(trans-5-Octyl-1,3-dioxan-2-yl)-phenyl-4-(6-cyclopentyl-hexyloxy)-benzoat;
4-(trans-5-Octyl-1,3-dioxan-2-yl)-phenyl-3-fluor-4-(6-cyclopentyl-hexyloxy)-benzoat;
4-(trans-5-Nonyl-1,3-dioxan-2-yl)-phenyl-3-fluor-4-(6-cyclopentylhexyloxy)-benzoat;
4-(trans-5-Nonyl-1,3-dioxan-2-yl)-phenyl-2,3-difluor-4-(6-cyclopentylhexyloxy)-benzoat;
4-(trans-5-Nonyl-1,3-dioxan-2-yl)-phenyl-2,3-difluor-4-(7-cyclopentylheptyloxy)-benzoat;
trans-5-Nonyl-2-[2',3'-difluor-4'-(7-cyclopentyl-heptyloxy)-biphenyl-4-yl]-1,3-dioxan;
5-(trans-5-Octyl-1,3-dioxan-2-yl)-[2-(3-fluor-4-(6-cyclopentyl-hexyloxy)-phenyl] -pyridin;
5-(trans-5-Nonyl-1,3-dioxan-2-yl)-[2-(2,3-difluor-4-(7-cyclopentylheptyloxy)-phenyl] -pyridin;
4'-[5-(5-Cyclopentyl-pentyloxy)-pyrimidin-2-yl]-phenyl-trans 4-hexylcyclohexan-carboxylat;
4'-[5-(5-Cyclopentyl-pentyloxy)-pyrimidin-2-yl]-phenyl-trans 4-pentylcyclohexan-carboxylat;
5-(5-Cyclopentyl-pentyloxy)-2[4-[2-(trans-4-hexyl-cyclohexyl)-ethyl]-phenyl]-pyrimidin;
5-(5-Cyclopentyl-pentyloxy)-2 [[2-(trans-4-hexyl-cyclohexyl)-methoxy]-phenyl] -pyrimidin;
5-(5-Cyclopentyl-pentyloxy)-2-[4-(trans-4-hexyl-cyclohexyl)-phenyl]-pyrimidin;
5-(7-Cyclopentyl-heptyloxy)-2-[4'-pentyl-biphenyl-4-yl]-pyrimidin;
5-(8-Cyclopentyl-octyloxy)-2-[4'-pentyl-biphenyl-4-yl]-pyrimidin;
5-(7-Cyclopentyl-heptyloxy)-2-[4'-hexyl-biphenyl-4-yl]-pyrimidin;
5-(4-Cyclopentyl-butyloxy)-2-[4'-hexyl-biphenyl-4-yl] -pyrimidin;
5-(6-Cyclopentyl-hexyloxy)-2- [4'-hexyl-biphenyl-4-yl] -pyrimidin;
5-(7-Cyclopentyl-heptyloxy)-2-[4'-hexyl-biphenyl-4-yl]-pyrimidin;
5-(8-Cyclopentyl-octyloxy)-2-[4'-hexyl-biphenyl-4-yl]-pyrimidin, Smp. (C-S_{C}) 119,0 °C, SC-N 157,0 °C, Klp. (N-I) 162,5 °C;
5-(6-Cyclopentyl-hexyloxy)-2-[4'-heptyl-biphenyl-4-yl]-pyrimidin;
5-(7-Cyclopentyl-heptyloxy)-2-[4'-heptyl-biphenyl-4-yl]-pyrimidin;
5-Heptyl-2-[4'-(6-cyclopentyl-hexyloxy)-biphenyl-4-yl]-pyrimidin;
5-Heptyl-2-[4'-(7-cyclopentyl-heptyloxy)-biphenyl-4-yl]-pyrimidin;
5-(7-Cyclopentyl-heptyloxy)-2-[4'-pentyl-biphenyl-4-yl]-pyrimidin;
5-(8-Cyclopentyl-octyloxy)-2-[4'-pentyl-biphenyl-4-yl]-pyrimidin;
2-(7-Cyclopentyl-heptyloxy)-5-[4'-hexyl-biphenyl-4-yl]-pyrimidin;
2-(4-Cyclopentyl-butyloxy)-5-[4'-hexyl-biphenyl-4-yl]-pyrimidin;
2-(6-Cyclopentyl-hexyloxy)-5-[4'-hexyl-biphenyl-4-yl]-pyrimidin;
2-(7-Cyclopentyl-heptyloxy)-5-[4'-hexyl-biphenyl-4-yl]-pyrimidin;
2-(8-Cyclopentyl-octyloxy)-5-[4'-hexyl-biphenyl-4-yl]-pyrimidin;
2-(6-Cyclopentyl-hexyloxy)-5-[4'-heptyl-biphenyl-4-yl]-pyrimidin;
2-(7-Cyclopentyl-heptyloxy)-5- [4'-heptyl-biphenyl-4-yl] -pyrimidin;
2-Heptyl-5-[4'-(6-cyclopentyl-hexyloxy)-biphenyl-4-yl]-pyrimidin;
2-Heptyl-5-[4'-(7-cyclopentyl-heptyloxy)-biphenyl-4-yl]-pyrimidin;
2-(4-Hexyl-phenyl)-5-[4-(7-cyclopentyl-heptyloxy)-phenyl]-pyrimidin;
2-(4-Heptyl-phenyl)-5-[4-(7-cyclopentyl-heptyloxy)-phenyl]-pyrimidin;
4-[2-[4-(7-Cyclopentyl-heptyloxy)-phenyl]-pyrimidin-5-yl]-phenyl-(R)-2-fluor-octanoat;
4-[2-[4-(7-Cyclopentyl-heptyloxy)-phenyl]-pyrimidin-5-yl]-phenyl-(R)-2-methyl-octanoat;
4-[2-[4-(7-Cyclopentyl-heptyloxy)-phenyl]-pyrimidin-5-yl]-phenyl-(R)-2-fluor-2-methyl-octanoat;
5-Heptyl-2-[6-(7-cyclopentyl-heptyloxy)-naphth-2-yl]-pyrimidin;
5-Octyl-2-[6-(7-cyclopentyl-heptyloxy)-naphth-2-yl]-pyrimidin;
5-Nonyl-2-[6-(7-cyclopentyl-heptyloxy)-naphth-2-yl]-pyrimidin;
6-Octyl-2-[4-(7-cyclopentyl-heptyloxy)-phenyl]-chinolin;
6-Nonyl-2-[4-(7-cyclopentyl-heptyloxy)-phenyl]-chinolin;
2-(4-Hexyl-phenyl)-5-[4-(5-cyclopentyl-pentyloxy)-phenyl]-1,3,4-thiadiazol;
2-(4-Hexyl-phenyl)-5-[4-(5-cyclopentyl-pentyloxy)-phenyl]-thiazol;
5-(4-Hexyl-phenyl)-2-[4-(5-cyclopentyl-pentyloxy)-phenyl]-thiazol;
2-(4-Hexyl-phenyl)-5-[4-(5-cyclopentyl-pentyloxy)-phenyl]-thiophen;
4'-(7-Cyclopentyl-heptyloxy)-biphenyl-4-carbonsäure-4-heptyl-phenyl-ester;
4'-(7-Cyclopentyl-heptyloxy)-biphenyl-4-carbonsäure-(S)-4-[1-(pentyloxycarbonyl)-ethyl]-phenyl-ester.

### Beispiel 2

Eine Lösung von 1,86 g 5-(9-Cyclopentyl-non-1-enyl)-2-(4-heptyloxyphenyl)-pyrimidin in 35 ml Toluol wird mit 0,24 g 5-%iger Palladiumkohle bei Raumtemperatur und Normaldruck bis zum Stillstand hydriert. Umkristallisation des nach Filtration und Einengen des Filtrats erhaltenen Rohprodukts aus Ethanol liefert reines 5-(9-Cyclopentyl-nonyl)-2-(4-heptyloxyphenyl)-pyrimidin.

Das als Ausgangsmaterial eingesetzte 5-(9-Cyclopentyl-non-1-enyl)-2-(4-heptyloxy-phenyl)-pyrimidin kann wie folgt hergestellt werden:
a) In Stickstoffatmosphäre tropft man bei 0 bis 5 °C innert 15 Minuten 11 ml 20-%ige (v/v) Diisobutylaluminiumhydrid-Lösung in Toluol zu einer Lösung von 2,09 g 2-(4-Heptyloxy-phenyl)-pyrimidin-5-carbonitril in 40 ml Toluol. Das Reaktionsgemisch wird noch 2 Stunden bei 0 °C gerührt und auf 100 ml eiskalte 1N Salzsäure gegossen. Nach beendeter Reaktion werden die Phasen getrennt. Die organische Phase wird mit Wasser neutral gewaschen, über Natriumsulfat getrocknet, filtriert und das Filtrat eingeengt. Man erhält 2-(4-Heptyloxy-phenyl)-pyrimidin-5-carboxaldehyd.
b) Eine Suspension von 3,7 g (8-Cyclopentyl-octyl)-triphenylphosphonium-bromid (hergestellt durch Erhitzen von 3,4 g (8-Bromoctyl)-cyclopentan und 3,9 g Triphenylphosphin in 15 ml Toluol über Nacht und Ausfällen des Produkts aus dem abgekühlten Reaktionsgemisch durch Zugabe von Hexan) in 20 ml tert.-Butyl-methyl-ether wird mit 0,8 g Kalium-tert.-butylat versetzt und die gelbe Suspension 45 Minuten bei Raumtemperatur gerührt. Dann wird bei 0 bis 5 °C eine Lösung von 1,64 g 2-(4-Heptyloxy-phenyl)-pyrimidin-5-carboxaldehyd in 10 ml Tetrahydrofuran zugetropft. Das Reaktionsgemisch wird noch 2 Stunden bei 0 °C gerührt und dann mit 10 ml gesättigter Natrium-hydrogencarbonat-Lösung verrührt. Die Phasen werden getrennt und die organische Phase mit gesättigter Kochsalz-Lösung gewaschen, über Natriumsulfat getrocknet, filtriert und eingeengt. Der Rückstand wird zwischen Methanol/Wasser (vol.) 4:1 und Hexan verteilt und die Methanol-Phase abgetrennt. Die Hexan-Phase wird mit Methanol/Wasser 4:1 gewaschen und das Filtrat eingeengt. Das erhaltene rohe 5-(9-Cyclopentyl-non-1-enyl)-2-(4-heptyloxy-phenyl)-pyrimidin wird zur Reinigung an 10 g Kieselgel mit Hexan/Ethylacetat 19:1 (v/v) chromatographiert.

Analog können folgende Verbindungen hergestellt werden:
5-(8-Cyclopentyl-octyl)-2-(4-heptyloxy-phenyl)-pyrimidin;
5-(8-Cyclopentyl-octyl)-2-(4-octyloxy-phenyl)-pyrimidin;
5-(8-Cyclopentyl-octyl)-2-(4-oct-2E-enyloxy-phenyl)-pyrimidin;
5-(8-Cyclopentyl-octyl)-2-(4-oct-3Z-enyloxy-phenyl)-pyrimidin;
5-(8-Cyclopentyl-octyl)-2-(4-oct-5Z-enyloxy-phenyl)-pyrimidin;
(R)-5-(6-Cyclopentyl-hexyl)-2-[4-(2-fluor-octyloxy)-phenyl]-pyrimidin;
(R)-5-(7-Cyclopentyl-heptyl)-2-[4-(2-fluor-octyloxy)-phenyl]-pyrimidin;
(R)-5-(8-Cyclopentyl-octyl)-2-[4-(2-fluor-octyloxy)-phenyl]-pyrimidin;
(R)-5-(9-Cyclopentyl-nonyl)-2-[4-(2-fluor-octyloxy)-phenyl]-pyrimidin;
(R)-5-(10-Cyclopentyl-decyl)-2-[4-(2-fluor-octyloxy)-phenyl]-pyrimidin;
(R)-5-(8-Cyclopentyl-octyl)-2-[3-fluor-4-(2-fluor-octyloxy)-phenyl]-pyrimidin;
(R)-5-(6-Cyclopentyl-hexyl)-2-[2,3-difluor-4-(2-fluor-octyloxy)-phenyl]-pyrimidin;
(R)-5-(7-Cyclopentyl-heptyl)-2-[2,3-difluor-4-(2-fluor-octyloxy)-phenyl]-pyrimidin;
(R)-5-(8-Cyclopentyl-octyl)-2-[2,3-difluor-4-(2-fluor-octyloxy)-phenyl]-pyrimidin;
(R)-5-(9-Cyclopentyl-nonyl)-2-[2,3-difluor-4-(2-fluor-octyloxy)-phenyl]-pyrimidin;
(S)-5-(7-Cyclopentyl-heptyl)-2-[4-(3-fluor-octyloxy)-phenyl]-pyrimidin;
(S)-5-(8-Cyclopentyl-octyl)-2-[4-(3-fluor-octyloxy)-phenyl]-pyrimidin;
(S,S)-5-(7-Cyclopentyl-heptyl)-2-[4-(2,3-difluor-octyloxy)-phenyl]-pyrimidin;
(S,S)-5-(8-Cyclopentyl-octyl)-2-[4-(2,3-difluor-octyloxy)-phenyl]-pyrimidin;
(S)-5-(8-Cyclopentyl-octyl)-2-[4-(3-fluor-octyloxy)-phenyl]-pyrimidin;
(R,R)-5-(8-Cyclopentyl-octyl)-2-[4-(2,3-epoxy-nonyloxy)-phenyl]-pyrimidin;
(R,R)-5-(9-Cyclopentyl-nonyl)-2-[4-(2,3-epoxy-nonyloxy)-phenyl]-pyrimidin;
5-(8-Cyclopentyl-octyl)-2-(4-octyloxy-phenyl)-pyrimidin;
5-(6-Cyclopentyl-hexyl)-2-(4-heptyloxy-phenyl)-pyridin;
5-(8-Cyclopentyl-octyl)-2-(4-heptyloxy-phenyl)-pyridin;
(R)-5-(8-Cyclopentyl-octyl)-2-[4-(2-fluor-octyloxy)-phenyl]-pyridin;
5-(8-Cyclopentyl-octyl)-2-(4-heptyloxy-phenyl)-pyrazin;
4-[5-(8-Cyclopentyl-octyl)-pyrimidin-2-yl]-phenyl-(R)-2-fluor-octanoat;
4-[5-(8-Cyclopentyl-octyl)-pyrimidin-2-yl]-phenyl-(R)-2-chlor-octanoat;
4-[5-(8-Cyclopentyl-octyl)-pyrimidin-2-yl]-phenyl-(S)-2-cyan-octanoat;
4-[5-(8-Cyclopentyl-octyl)-pyrimidin-2-yl]-phenyl-(S)-2-methyl-octanoat;
4-[5-(8-Cyclopentyl-octyl)-pyrimidin-2-yl]-phenyl-(R)-2-(trifluormethyl)-octanoat;
4-[5-(8-Cyclopentyl-octyl)-pyrimidin-2-yl]-phenyl-(R)-2-fluor-2-methyl-octanoat;
4-[5-(8-Cyclopentyl-octyl)-pyrimidin-2-yl]-phenyl-(2R,3S)-2,3-epoxy-octanoat;
4-[5-[4-(6-Cyclopentyl-hexyl)-phenyl]-pyrimidin-2-yl]-phenyl-(S)-2-chlor-3-methyl-butanoat;
4-[5-[4-(6-Cyclopentyl-hexyl)-phenyl]-pyrimidin-2-yl]-phenyl-(S,S)-2-chlor-3-methyl-pentanoat;
4-[5-[4-(6-Cyclopentyl-hexyl)-phenyl]-pyrimidin-2-yl]-benzoesäure-(S)-oct-2-yl-ester;
4-[5-[4-(6-Cyclopentyl-hexyl)-phenyl]-pyrimidin-2-yl]-benzoesäure-(R)-1-(trifluormethyl)-heptyl-ester;
4-[5-[4-(6-Cyclopentyl-hexyl)-phenyl]-pyrimidin-2-yl]-benzoesäure-(S)-2-butyloxy-propyl-ester;
4-[5-[4-(6-Cyclopentyl-hexyl)-phenyl]-pyrimidin-2-yl]-benzoesäure-(S)-2-pentyloxy-propyl-ester;
(S)-5-[4-(6-Cyclopentyl-hexyl)-phenyl]-2-(4-oct-2-yloxy-phenyl)-pyrimidin;
(S)-2-[4-[5-[4-(6-Cyclopentyl-hexyl)-phenyl]-pyrimidin-2-yl]-phenoxy]-propionsäure-pentyl-ester.

### Beispiel 3

Eine Lösung von 0,24 g N,N'-Dicyclohexyl-carbodiimid in 5 ml Dichlormethan wird innert 15 Minuten bei 0 °C zu einer Lösung von 0,125 g Cyclopentan-nonansäure, 0,142 g 4-(5-Octyl-pyrimidin-2-yl)-phenol und 0,015 g 4-Dimethylamino-pyridin in 10 ml Dichlormethan getropft. Die Suspension wird noch 30 Minuten bei 0 °C gerührt, filtriert und das Filtrat eingeengt. Der Rückstand wird an 10 g Kieselgel mit Hexan/Ethylacetat 19:1 (v/v) chromatographiert. Umkristallisation der produkthaltigen Fraktionen aus Ethanol liefert reines 4-(5-Octyl-pyrimidin-2-yl)-phenyl-cyclopentan-nonanoat.

Analog können folgende Verbindungen hergestellt werden:
4-(5-Octyl-pyrimidin-2-yl)-phenyl-cyclopentan-octanoat;
4-(5-Nonyl-pyrimidin-2-yl)-phenyl-cyclopentan-heptanoat;
4-(5-Nonyl-pyrimidin-2-yl)-phenyl-cyclopentan-octanoat;
4-(5-Nonyl-pyrimidin-2-yl)-phenyl-cyclopentan-nonanoat;
(R)-4-[5-(2-Fluor-octyl)-pyrimidin-2-yl]-phenyl-cyclopentan-nonanoat;
4-(5-Heptyl-pyridin-2-yl)-phenyl-cyclopentan-heptanoat;
4-(5-Octyl-pyridin-2-yl)-phenyl-cyclopentan-octanoat;
4-(5-Nonyl-pyridin-2-yl)-phenyl-cyclopentan-heptanoat;
4'-(5-Nonyl-pyridin-2-yl)-biphenyl-4-yl-cyclopentan-hexanoat;
4'-(5-Octyl-pyridin-2-yl)-biphenyl-4-yl-cyclopentan-octanoat;
4'-(5-Decyl-pyridin-2-yl)-biphenyl-4-yl-cyclopentan-octanoat;
4'-(5-Hexyl-pyrimidin-2-yl)-biphenyl-4-yl-cyclopentan-hexanoat;
4'-(5-Pentyl-pyrimidin-2-yl)-biphenyl-4-yl-cyclopentan-octanoat;
4'-(5-Hexyl-pyrimidin-2-yl)-biphenyl-4-yl-cyclopentan-octanoat;
4'-(5-Heptyl-pyrimidin-2-yl)-biphenyl-4-yl-cyclopentan-octanoat;
4'-(5-Pentyl-pyrimidin-2-yl)-biphenyl-4-yl-cyclopentan-nonanoat;
4'-(5-Hexyl-pyrimidin-2-yl)-biphenyl-4-yl-cyclopentan-nonanoat;
4'-(5-Heptyl-pyrimidin-2-yl)-biphenyl-4-yl-cyclopentan-nonanoat;
4'-(5-Pentyl-pyrimidin-2-yl)-biphenyl-4-yl-cyclopentan-decanoat;
2-(4'-Pentyl-biphenyl-4-yl)-pyrimidin-5-yl-cyclopentan-heptanoat;
2-(4'-Hexyl-biphenyl-4-yl)-pyrimidin-5-yl-cyclopentan-hexanoat;
2-(4'-Hexyl-biphenyl-4-yl)-pyrimidin-5-yl-cyclopentan-heptanoat;
2-(4'-Heptyl-biphenyl-4-yl)-pyrimidin-5-yl-cyclopentan-hexanoat.
2-(4'-Heptyl-biphenyl-4-yl)-pyrimidin-5-yl-cyclopentan-heptanoat;
2-(4'-Pentyl-biphenyl-4-yl)-pyrimidin-5-yl-cyclopentan-octanoat;
2-(4'-Hexyl-biphenyl-4-yl)-pyrimidin-5-yl-cyclopentan-octanoat;
2-(4'-Heptyl-biphenyl-4-yl)-pyrimidin-5-yl-cyclopentan-octanoat;
2-(4'-Pentyl-biphenyl-4-yl)-pyrimidin-5-yl-cyclopentan-nonanoat;
2-(4'-Hexyl-biphenyl-4-yl)-pyrimidin-5-yl-cyclopentan-nonanoat;
(R)-2-[4'-(2-Fluor-octyl)-biphenyl-4-yl]-pyrimidin-2-yl)-cyclopentannonanoat;
(S)-2-[4'-(3-Fluor-nonyl}-biphenyl-4-yl]-pyrimidin-2-yl)-cyclopentannonanoat;
4-[[4-(trans-4-Heptyl-cyclohexyl)-phenyl] -ethinyl] -phenyl-cyclopentanheptanoat;
4"-Heptyl-2'-fluor-[1,1';4'-1"]terphenyl-4-yl-cyclopentan-heptanoat;
4"-Heptyl-3-fluor-[1,1';4'-1"]terphenyl-4-yl-cyclopentan-hexanoat;;
4"-Hexyl-2",3"-difluor-[1,1';4'-1"]terphenyl-4-yl-cyclopentan-octanoat;
4"-Heptyl-2,3-difluor-[1,1';4'-1"]terphenyl-4-yl-cyclopentan-heptanoat;
4"-Hexyl--3,2"-difluor-[1,1';4'-1"]terphenyl-4-yl-cyclopentan-octanoat.

### Beispiel 4

Eine Lösung von 0,3 g N,N'-Dicyclohexyl-carbodiimid in 6 ml Dichlormethan wird innert 15 Minuten bei 0 °C zu einer Lösung von 0,201 g 4-(5-Octyl-pyrimidin-2-yl)-benzoesäure, 0,116 g Cyclopentan-hexanol und 0,02 g 4-Dimethylamino-pyridin in 10 ml Dichlormethan getropft. Die Suspension wird noch 1 Stunde bei 0 °C gerührt, filtriert und eingeengt. Der Rückstand wird an 10 g Kieselgel mit Hexan/Ethylacetat 19:1 (v/v) chromatographiert. Umkristallisation der produkthaltigen Fraktionen aus Ethanol ergibt reinen 4-(5-Octyl-pyrimidin-2-yl)-benzoesäure-6-cyclopentyl-hexyl-ester.

Analog können folgende Verbindungen hergestellt werden:
4-(5-Nonyl-pyrimidin-2-yl)-benzoesäure-6-cyclopentyl-hexyl-ester;
4-(5-Octyl-pyrimidin-2-yl)-benzoesäure-7-cyclopentyl-heptyl-ester;
4-(5-Hepyl-pyrimidin-2-yl)-benzoesäure-7-cyclopentyl-heptyl-ester;
4-(5-Nonyl-pyrimidin-2-yl)-benzoesäure-7-cyclopentyl-heptyl-ester;
4-(5-Nonyl-pyrimidin-2-yl)-2-fluor-benzoesäure-6-cyclopentyl-hexyl-ester;
4-(5-Octyl-pyrimidin-2-yl)-2,3-difluor-benzoesäure-6-cyclopentyl-hexylester;
4-(5-Hepyloxy-pyrimidin-2-yl)-benzoesäure-6-cyclopentyl-hexyl-ester;
4-(5-Nonyl-pyridin-2-yl)-benzoesäure-6-cyclopentyl-hexyl-ester;
4-(5-Octyl-pyridin-2-yl)-benzoesäure-7-cyclopentyl-heptyl-ester;
4-(5-Nonyl-pyrazin-2-yl)-benzoesäure-6-cyclopentyl-hexyl-ester;
4-(5-Octyl-pyrazin-2-yl)-benzoesäure-7-cyclopentyl-heptyl-ester;
4-(4-Nonyl-benzoyloxy)-benzoesäure-8-cyclopentyl-octyl-ester;
4-(4-Octyloxy-benzoyloxy)-benzoesäure-8-cyclopentyl-octyl-ester.

### Beispiel 5

Zur Untersuchung der Eigenschaften von Verbindungen der Formel I wurden zu 85 Gew.-% einer Grundmischung (GM) jeweils 15 Gew.-% einer Verbindung der Formel I bzw. zu Vergleichszwecken 15 Gew.% einer analogen Verbindung ohne Cyclopentan-Ring zugemischt. Von diesen Mischungen wurden die Phasenfolge bestimmt sowie die spontane Polarisation (P_{S}), die Schaltzeit (τ) und teilweise der Schaltwinkel (2θ) gemessen. Die Messungen wurden unter den folgenden Bedingungen durchgeführt: P_{S} bei 8,5 µm Zelldicke und einer Dreieckspannung von 10 Hz und 5 V/µm; Schaltzeiten (bis Imax) bei 10 V_{pp/µm} Rechteckspannung; Schaltwinkel bei 2 µm Zelldicke und einer Spannung von 25 V. Alle Messungen wurden bei 25 °C durchgeführt.

Grundmischung (GM)
- 16,6 Gew.-%: trans-4-[4-(2,3-Difluor-4-octyloxy-benzoyloxy)-phenyl]-cyclohexyl-(R)-2-fluorhexanoat,
- 23,8 Gew.-%: 5-Nonyl-2-(4-hexyloxy-phenyl)-pyrimidin,
- 23,4 Gew.-%: 5-Nonyl-2-(4-nonyloxy-phenyl)-pyrimidin,
- 11,8 Gew.-%: 5-Octyl-2-(4-nonyloxy-phenyl)-pyrimidin,
- 12,3 Gew.-%: 5-Octyl-2-(4-decyloxy-phenyl)-pyrimidin,
- 12,1 Gew.-%: 5-Heptyl-2-(4-heptyloxy-phenyl)-pyrimidin:
Phasenfolge [°C] I 74,6 N* 67,7 S_{A} 60,6 S_{C}*.

### Mischung 1

- 85 Gew.-%: GM
- 15 Gew.-%: 5-Nonyl-2-[4-(7-cyclopentyl-heptyloxy)-phenyl]-pyrimidin:
Phasenfolge I 74,6 N* 67,7 S_{A} 60,6 S_{C}*; Pₛ 15,8nC/cm²; τ 174 µs; 2θ 53,9°.

### Vergleichsmischung 1

- 85 Gew.-%: GM mit
- 15 Gew.-%: 5-Nonyl-2-(4-nonyloxy-phenyl)-pyrimidin:
Phasenfolge I 71,0 N* 66,0 S_{A} 60,0 S_{C}*; Pₛ 16,0 nC/cm²; τ 120 µs; 2θ 50,2°.

Die Mischung 1 führt gegenüber der Vergleichsmischung 1, bei sonst ähnlichen physikalischen Daten,zwar zu einer längeren Schaltzeit, dafür aber zu einem deutlich grösseren Schaltwinkel.

### Mischung 2

- 85 Gew.-%: GM mit
- 15 Gew.-%: 5-Octyl-2-[4-(8-cyclopentyl-octyloxy)-phenyl]-pyrimidin:
Phasenfolge I 72,2 N* 64,4 S_{A} 58,3 S_{C}*; Pₛ 17,0 nC/cm²; τ 150 µs.

### Mischung 3

- 85 Gew.-%: GM mit
- 15 Gew.-%: 5-Nonyl-2-[4-(8-cyclopentyl-octyloxy)-phenyl]-pyrimidin:
Phasenfolge I 72,5 N* 66,5 S_{A} 58,9 S_{C}*; Pₛ 15,5 nC/cm²; τ 140 µs.

### Mischung 4

- 85 Gew.-%: GM mit
- 15 Gew.-%: 5-Nonyloxy-2-[4-(7-cyclopentyl-heptyloxy)-phenyl]-pyrimidin:
Phasenfolge I 76,9 N* 71,9 S_{A} 63,5 S_{C}*; Pₛ 16,5 nC/cm²; τ 153 µs.

### Mischung 5

- 85 Gew.-%: GM mit
- 15 Gew.-%: 5-Nonyloxy-2-[4-(8-cyclopentyl-octyloxy)-phenyl]-pyrimidin:
Phasenfolge I 76,7 N* 70,6 S_{A} 61,8 S_{C}*; Pₛ 16,1 nC/cm²; τ 150 µs.

### Mischung 6

- 85 Gew.-%: GM mit
- 15 Gew.-%: 5-Decyloxy-2-[4-(8-cyclopentyl-octyloxy)-phenyl]-pyrimidin:
Phasenfolge I 76,5 N* 71,1 S_{A} 61,2 S_{C}*; Pₛ 16,0 nC/cm²; τ 132 µs.

### Mischung 7

- 85 Gew.-%: GM mit
- 15 Gew.-%: 5-(7-Cyclopentyl-heptyloxy)-2-(4-octyloxy-phenyl)-pyrimidin:
Phasenfolge I 77,7 N* 73,8 S_{A} 65,9 S_{C}*; Pₛ 16,6nC/cm²; τ 140 µs; 2θ 54°.

### Vergleichsmischung 2

- 85 Gew.-%: GM mit
- 15 Gew.-%: 5-Oct-7-enyloxy-2-(4-oct-7-enyloxy-phenyl)-pyrimidin:
Phasenfolge I 74,7 N* 69,7 S_{A} 58,2 S_{C}*; Pₛ 14,8nC/cm²; τ 90 µs; 2θ 49,4°.

Die Mischung 7 führt zu einer längeren Schaltzeit, dafür aber zu einem deutlich grösseren Schaltwinkel und zu einer deutlich höheren S_{C}*-Obergrenze als die Vergleichsmischung 2.

### Mischung 8

- 85 Gew.-%: GM mit
- 15 Gew.-%: 5-(8-Cyclopentyl-octyloxy)-2-[4-(8-cyclopentyl-octyloxy)-phenyl] -pyrimidin:
Phasenfolge I 75,5 N* 73,5 S_{A} 60,9 S_{C}*; Pₛ 15,8 nC/cm²; τ 155 µs.

### Mischung 9

- 85 Gew.-%: GM mit
- 15 Gew.-%: 5-Nonyl-2-[4-(7-cyclopentyl-heptyloxy)-phenyl]-pyridin:
Phasenfolge I 73,0 N* 69,2 S_{A} 63.4 S_{C}*; Pₛ 17,1nC/cm²; τ 136 µs; 2θ 54,3°.

### Vergleichsmischung 3

- 85 Gew.-%: GM mit
- 15 Gew.-%: 5-Nonyl-2-(4-dec-9-enyloxy-phenyl)-pyridin:
Phasenfolge I 73,4 N* 70,3 S_{A} 61,6 S_{C}*; Pₛ 16,1 nC/cm²; τ 90 µs; 2θ 49,4°.

Die Mischung 9 führt zu einer längeren Schaltzeit, dafür aber zu einem deutlich grösseren Schaltwinkel und zu einer etwas höheren S_{C}∗-Obergrenze als die Vergleichsmischung 3.

### Mischung 10

- 85 Gew.-%: GM mit
- 15 Gew.-%: 5-Heptyl-2-[4-(8-cyclopentyl-octyloxy)-phenyl]-pyridin:
Phasenfolge I 73,0 N* 69,5 S_{A} 62,2 S_{C}*; Pₛ 16,1 nC/cm²; τ 130 µs.

### Mischung 11

- 85 Gew.-%: GM mit
- 15 Gew.-%: 5-Nonyl-2-[4-(8-cyclopentyl-octyloxy)-phenyl]-pyridin: Phasenfolge
I 72,1 N* 67,5 S_{A} 61,7 S_{C}*; Pₛ 16,9 nC/cm²; τ 136 µs.

## Patentansprüche

1. Cyclopentyl-Verbindungen der allgemeinen Formel 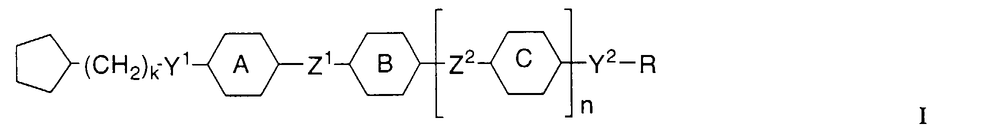 worin
k eine ganze Zahl von 4 bis 18;
n 0 oder 1;
Y¹ Y² eine Einfachbindung, -O-, -COO- oder -OOC-;
die Ringe A, B, C unabhängig voneinander gegebenenfalls mono- oder difluoriertes 1,4-Phenylen, Pyrimidin-2,5-diyl, Pyridin-2,5-diyl, Pyrazin-2,5-diyl, Naphthalin-2,6-diyl, Chinolin-2,6-diyl, Thiophen-2,5-diyl, Thiazol-2,5-diyl oder 1,3,4-Thiadiazol-2,5-diyl, und Ring C auch trans-1,4-Cyclohexylen oder trans-1,3-Dioxan-2,5-diyl;
Z¹ eine Einfachbindung, -COO-, -OOC- oder -C≡C-;
Z² eine Einfachbindung, -COO-, -OOC-, -OCH₂-, -CH₂O- oder -(CH₂)₂-; und
R geradkettiges oder verzweigtes, gegebenenfalls optisch aktives, Alkyl oder Alkenyl mit 4 bis 20 Kohlenstoffatomen, worin eine oder zwei nicht benachbarte Methylen-Gruppen durch -O-, -COO-, -OOC- und/oder Epoxyethylen und/oder mindestens ein Wasserstoff durch Fluor und/oder ein Wasserstoff durch Chlor oder Cyan und/oder ein endständiger Wasserstoff durch Cyclopentyl ersetzt sein können, bedeuten.

2. Verbindungen gemäss Anspruch 1, dadurch gekennzeichnet, dass die Ringe A, B, und C unabhängig voneinander 1,4-Phenylen, 2- bzw. 3-Fluor-1,4-phenylen, 2,3-Difluor-1,4-phenylen, Pyrimidin-2,5-diyl, Pyridin-2,5-diyl oder Pyrazin-2,5-diyl und Ring C auch trans-1,4-Cyclohexylen oder trans-1,3-Dioxan-2,5-diyl bedeutet.

3. Verbindungen nach einem der Ansprüche 1 oder 2, dadurch gekennzeichnet, dass mindestens einer der Ringe A, B oder C 1,4-Phenylen, 2-bzw. 3-Fluor-1,4-phenylen oder 2,3-Difluor-1,4-phenylen bedeutet und höchstens einer der Ringe A, B oder C Pyrimidin-2,5-diyl, Pyridin-2,5-diyl oder Pyrazin-2,5-diyl bedeutet.

4. Verbindungen der Formeln 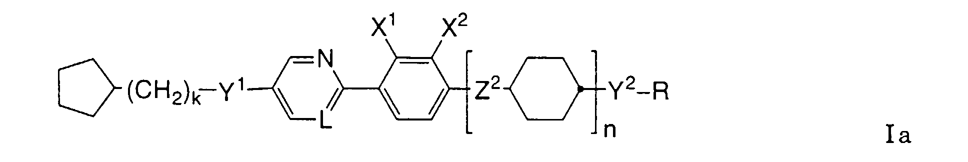 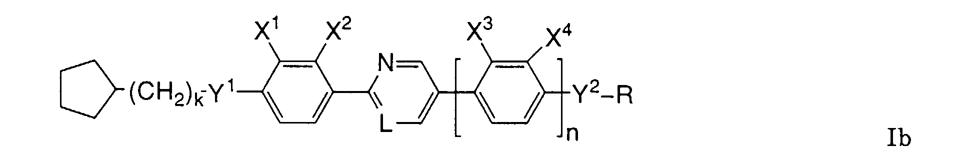 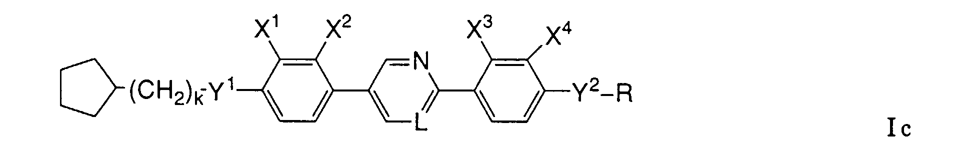 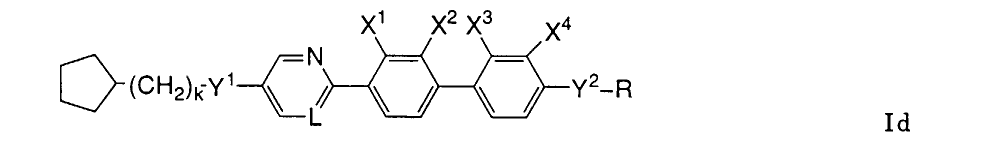 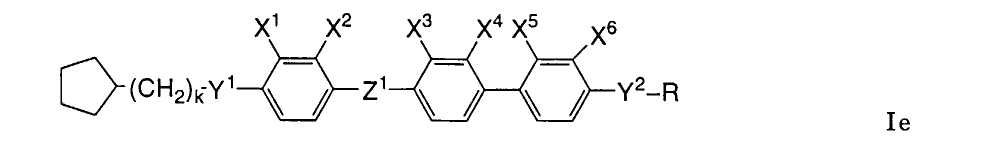 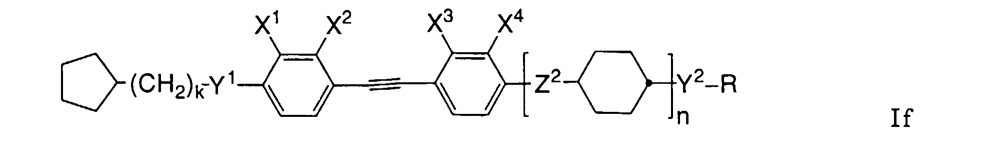 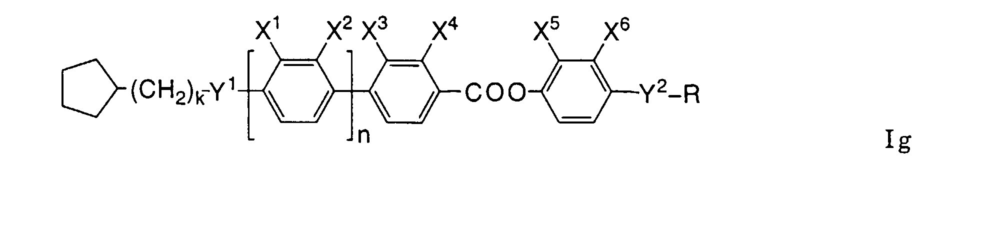 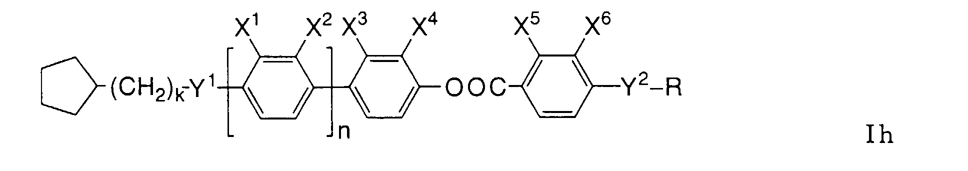 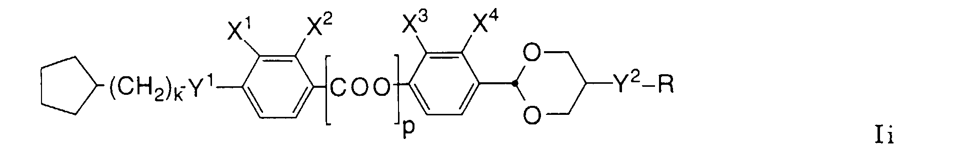 worin
L Stickstoff oder -CH=;
X¹⁻⁶ Wasserstoff oder Fluor;
p 0 oder 1 bedeuten; und
n, k, Y¹, Y² und R die Anspruch 1 angegebenen Bedeutungen haben.

5. Verbindungen nach einem der Ansprüche 1 bis 4, dadurch gekennzeichnet, dass k eine ganze Zahl von 5 bis 12 bedeutet.

6. Verbindungen nach einem der Ansprüche 1 bis 5, dadurch gekennzeichnet, dass Y¹ eine Einfachbindung, -O- oder -COO- bedeutet.

7. Verbindungen nach einem der Ansprüche 1 bis 6, dadurch gekennzeichnet, dass Y¹ -O- bedeutet.

8. Verbindungen nach einem der Ansprüche 1 bis 7, dadurch gekennzeichnet, dass Rest R ein Alkyl- oder Alkenyl-Rest mit 5 bis 12 Kohlenstoffatomen, worin 1 bis 2 nicht endständige Methylengruppen durch -C*H(W)-, -C*F(CH₃)- und/oder Epoxyethylen ersetzt sind und 1 oder 2 nicht benachbarte Methylengruppen durch -O-, -COO-, -OOC- ersetzt sein können; W Fluor, Chlor, Cyan, Methyl oder Trifluormethyl und C* ein chirales Zentrum bedeuten.

9. Verbindungen nach einem der Ansprüche 1 bis 8, dadurch gekennzeichnet, dass die Gruppe -Y²-R eine optisch aktive Gruppe wie 2- oder 3-Fluoralkyl, 2- oder 3-Fluoralkoxy. 2,3-Difluoralkoxy, 2- oder 3-Fluor-alkanoyloxy, 2,3-Difluor-alkanoyloxy, 2-Fluor-2-methyl-alkanoyloxy, 2-Fluor-3-methyl-alkanoyloxy, 2- oder 3-Chlor-alkoxy, 2- oder 3-Chlor-alkanoyloxy, 2-Chlor-3-methyl-alkanoyloxy, 1- oder 2-Cyanalkyl, 1- oder 2-Cyanalkoxy, 2- oder 3-Cyan-alkanoyloxy, 1-, 2- oder 3-Methylalkyl, 1-, 2- oder 3-Methylalkoxy, 2- oder 3-Methyl-alkanoyloxy, 1-, 2- oder 3-Trifluormethyl-alkanoyloxy, 1,2-Epoxyalkyl, 2,3-Epoxyalkoxy, 2,3-Epoxy-alkanoyloxy, 1-Alkoxycarbonyl-ethyl, 1-Alkoxycarbonyl-ethoxy, 2-Alkoxy-propanoyloxy, (1-Methyl-alkoxy)-carbonyl, (1-Trifluormethyl-alkoxy)-carbonyl, 1-Alkoxy-2,2,2-trifluorethyl, ω-Trifluormethyl-ω-alkoxyalkyl mit jeweils 5 bis 12 Kohlenstoffatomen bedeutet.

10. Verbindungen nach einem der Ansprüche 1 bis 7, dadurch gekennzeichnet, dass Y² eine Einfachbindung, -O- oder -OOC- ; und der Rest R ein geradkettiger oder verzweigter (racemischer) Alkyl- oder Alkenyl-Rest mit 5 bis 12 Kohlenstoffatomen bedeutet, worin eine nicht mit Y² benachbarte Methylen-Gruppe durch -O-, -COO- oder -OOC- und/oder ein oder mehrere Wasserstoffatome durch Fluor und/oder ein endständiges Wasserstoffatom durch Cyclopentyl ersetzt sein kann.

11. Verbindungen nach einem der Ansprüche 1 bis 7 oder 10, dadurch gekennzeichnet, dass der Rest R einen geradkettigen oder methyl-verzweigten Alkyl- oder Alkenyl-Rest mit 5 bis 12 Kohlenstoffatomen bedeutet.

12. Flüssigkristalline Mischung enthaltend eine oder mehrere Verbindung gemäss einem der Ansprüche 1 bis 11.

13. Flüssigkristalline Mischung gemäss Anspruch 12, dadurch gekennzeichnet, dass als weitere Komponenten eine oder mehrere Verbindungen der Formeln 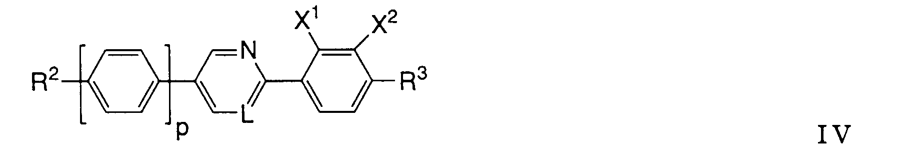 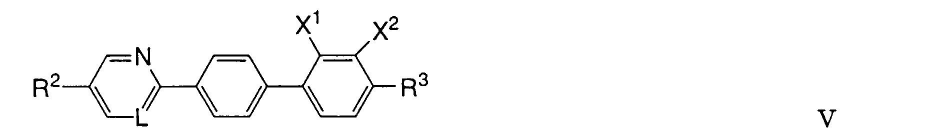 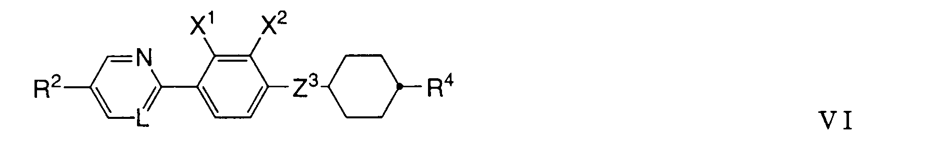 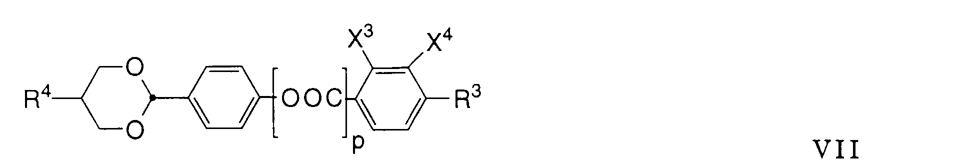 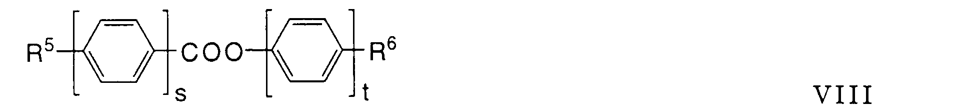 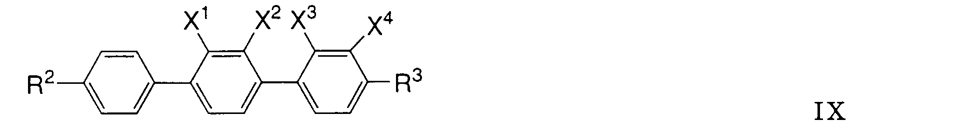 worin
p 0 oder 1;
s, t 1 oder 2 ist, wobei s + t = 2 oder 3;
L Stickstoff oder -CH=;
X¹, X², X³, X⁴ unabhängig voneinander Wasserstoff oder Fluor;
Z³ eine Einfachbindung, -OOC-, -OCH₂- oder -(CH₂)₂-;
R², R³ unabhängig voneinander Alkyl, Alkenyl, Alkoxy, Alkoxyalkyl, Alkenyloxy, Alkanoyloxy, Alkenoyloxy, Alkoxyalkoxy, Alkoxycarbonyl oder Alkenyloxycarbonyl;
R⁴ Alkyl oder Alkenyl;
R⁵, R⁶ unabhängig voneinander Alkyl, Alkenyl, Alkoxy oder Alkenyloxy bedeuten.
enthalten sind.

14. Flüssigkristalline Mischung gemäss einem der Ansprüche 12 oder 13, dadurch gekennzeichnet, dass eine oder mehrere optisch aktive Verbindungen aus der Gruppe der Verbindungen der allgemeinen Formeln 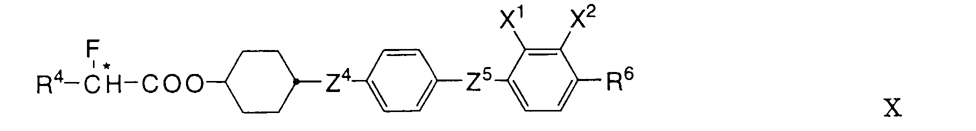 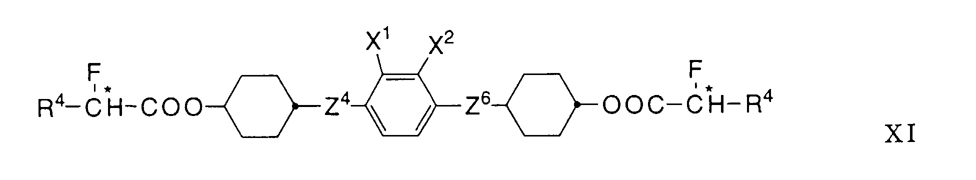 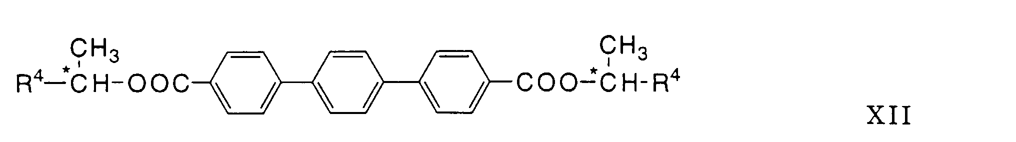 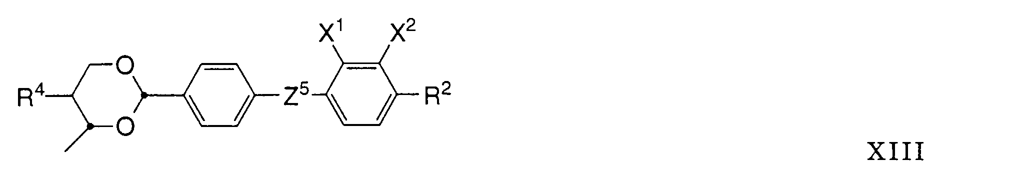 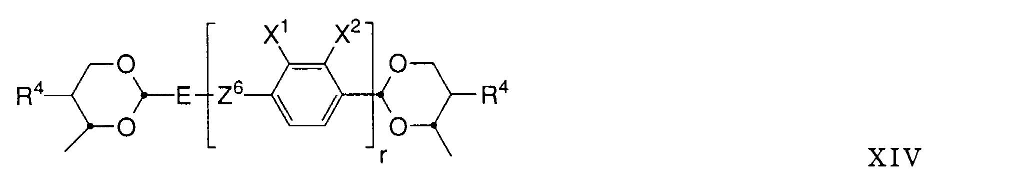 worin
E 1,4-Phenylen oder trans-1,4-Cyclohexylen;
r 0, 1 oder 2;
Z⁴ eine Einfachbindung, -(CH₂)₂- oder -CH₂O-;
Z⁵ eine Einfachbindung, -OCH₂-, -COO- oder -OOC-;
Z⁶ eine Einfachbindung, -(CH₂)₂- oder -OCH₂- bedeuten;
und die übrigen Symbole die in Anspruch 12 genannten Bedeutungen haben, enthalten sind.

15. Verwendung von flüssigkristallinen Verbindungen gemäss einem der Ansprüche 1 bis 10 für optische und elektro-optische Vorrichtungen.

16. Verwendung von flüssigkristallinen Mischungen gemäss einem der Ansprüche 11 bis 14 für optische und elektro-optische Vorrichtungen.

17. Optische und elektro-optische Vorrichtungen gemäss einem der Ansprüche 1 und 15 auf der Basis des DHF-, SBF- oder SSF-Effekts.

## Claims

1. Cyclopentyl compounds having the general formula: 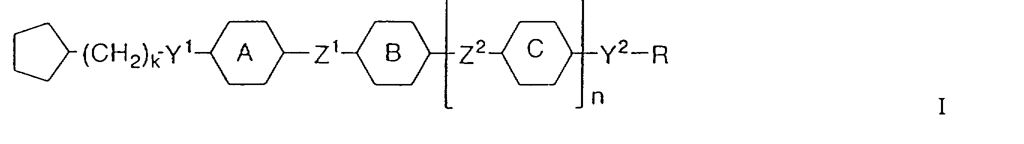 wherein
k is an integer from 4 to 18;
n is 0 or 1;
Y¹,Y² denote a single bond, -O-, -COO- or -OOC-;
the rings A, B, C independently of one another and as required denote mono- or difluorinated 1,4-phenylene, pyrimidin-2,5-diyl, pyridin-2,5-diyl, pyrazin-2,5-diyl, naphthalin-2,6-diyl, quinolin-2,6-diyl, thiophen-2,5-diyl, thiazole-2,5-diyl or 1,3,4-thiadiazole-2,5-diyl and ring C also denotes trans-1,4-cyclohexylene or trans-1,3-dioxan-2,5-diyl;
Z¹ denotes a single bond, -COO-, -OOC- or -C≡C-;
Z² denotes a single bond -COO-, -OOC-, -OCH₂-, -CH₂O-, or -(CH₂)₂- and
R is a straight-chain or branched, optionally optically active alkyl or alkenyl with 4 to 20 carbon atoms, wherein one or two non-neighbouring methylene groups can be replaced by -O-, -COO-, -OOC- and/or epoxyethylene and/or at least one hydrogen atom can be replaced by fluorine and/or one hydrogen atom can be replaced by chlorine or cyanogen and/or a terminal hydrogen atom can be replaced by cyclopentyl.

2. Compounds according to claim 1, characterised in that the rings A, B and C independently denote 1,4-phenylene, 2 or 3-fluoro-1,4-phenylene, 2,3-difluoro-1,4-phenylene, pyrimidin-2,5-diyl, pyridin-2,5-diyl or pyrazin-2,5-diyl and ring C also denotes trans-1,4-cyclohexylene or trans-1,3-dioxan-2,5-diyl.

3. Compounds according to claim 1 or 2, characterised in that at least one of the rings A, B or C denotes 1,4-phenylene, 2- or 3-fluoro-1,4-phenylene or 2,3-difluoro-1,4-phenylene and one at most of the rings A, B or C denotes pyrimidin-2,5-diyl, pyridin-2,5-diyl or pyrazin-2,5-diyl.

4. Compounds having the formulae: 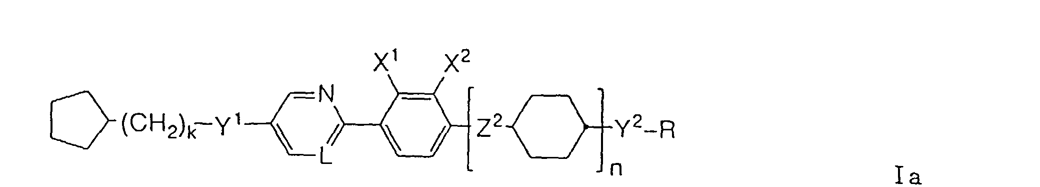 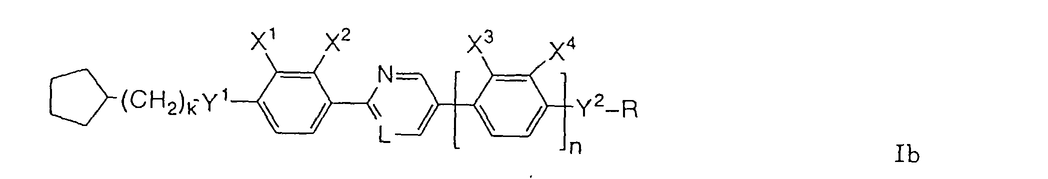 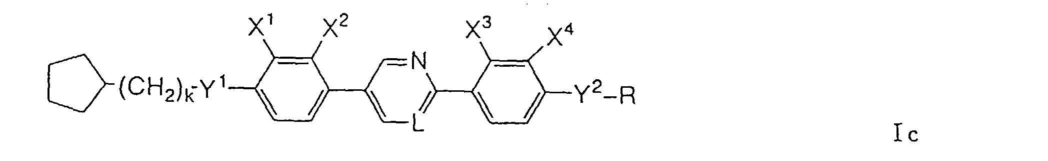 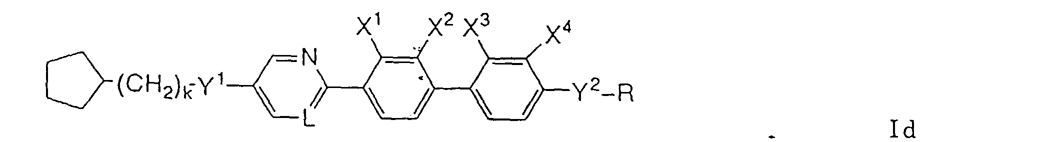 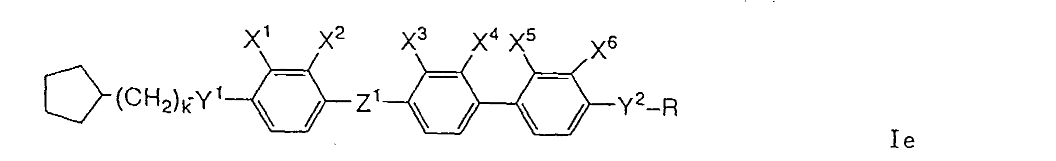 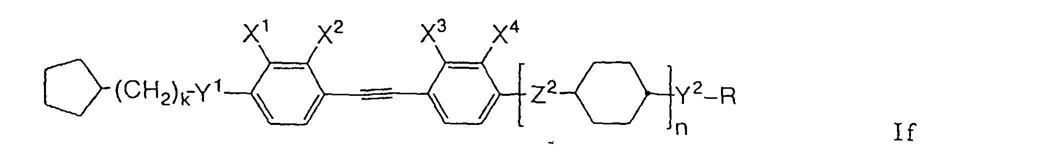 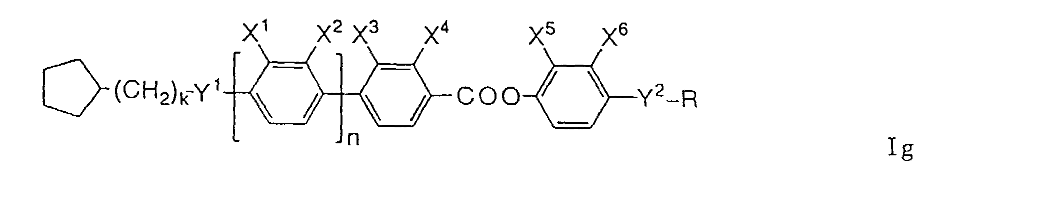 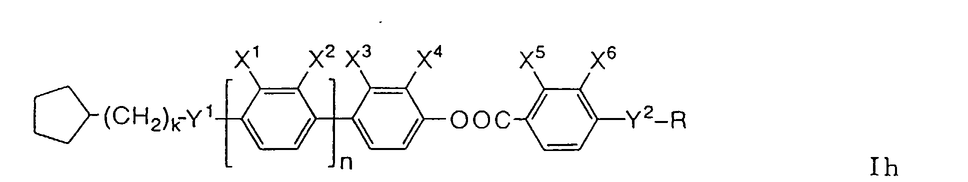 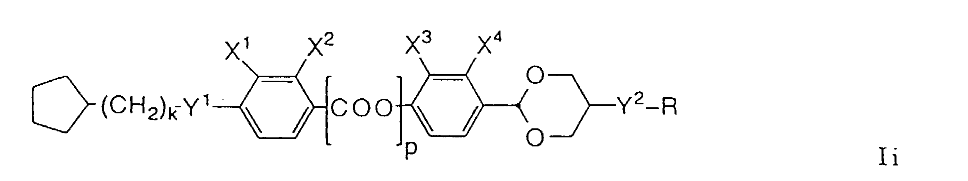 wherein
L denotes nitrogen or -CH=;
X¹⁻⁶ denotes hydrogen or fluorine;
P is 0 or 1 and
n, k, Y¹, Y² and R have the meanings given in claim 1.

5. Compounds according to any of claims 1 to 4, characterised in that k denotes an integer from 5 to 12.

6. Compounds according to any of claims 1 to 5, characterised in that Y¹ denotes a single bond, -O- or -COO-.

7. Compounds according to any of claims 1 to 6, characterised in that Y¹ denotes -O-.

8. Compounds according to any of claims 1 to 7, characterised in that the radical R denotes an alkyl or alkenyl radical with 5 to 12 carbon atoms wherein 1 or 2 non-terminal methylene groups are replaced by -C*H(W)-, -C*F(CH₃)- and/or epoxyethylene and 1 or 2 non-neighbouring methylene groups can be replaced by -O-, -COO-, -OOC-; W denotes fluorine, chlorine, cyanogen, methyl or trifluoromethyl and C* denotes a chiral centre.

9. Compounds according to any of claims 1 to 8, characterised in that the group -Y²-R denotes an optically active group such as 2- or 3-fluoroalkyl, 2,3-difluoroalkoxy, 2,3-difluoroalkoxy, 2- or 3-fluoroalkanoyloxy, 2,3-difluoro-alkanoyloxy, 2-fluoro-2-methyl-alkanoyloxy, 2-fluoro-3-methylalkanoyloxy, 2- or 3-chloroalkoxy, 2- or 3-chloroalkanoyloxy, 2-chloro-3-methyl-alkanoyloxy, 1- or 2-cyanoalkyl, 1- or 2-cyanoalkoxy, 2- or 3-cyanoalkanoyloxy, 1-, 2- or 3-methylalkyl, 1-, 2- or 3-methylalkoxy, 2- or 3-methyl-alkanoyloxy, 1-, 2- or 3-trifluoromethyl-alkanoyloxy, 1,2-epoxyalkyl, 2,3-epoxyalkoxy, 2,3-epoxy-alkanoyloxy, 1-alkoxycarbonyl-ethyl, 1-alkoxycarbonyl-ethoxy, 2-alkoxy-propanoyloxy, (1-methyl-alkoxy)-carbonyl, (1-trifluoromethyl-alkoxy)-carbonyl, 1-alkoxy-2,2,2-trifluoroethyl or ω-trifluoromethyl-ω-alkoxyalkyl, in each case with 5 to 12 carbon atoms.

10. Compounds according to any of claims 1 to 7, characterised in that y² denotes a single bond, -O- or -OOC-; and the radical R denotes a straight-chain or branched (racemic) alkyl or alkenyl radical with 5 to 12 carbon atoms, wherein a methylene group not neighbouring Y² can be replaced by -O-, -COO- or-OOC- and/or one or more hydrogen atoms can be replaced by fluorine and/or a terminal hydrogen atom can be replaced by cyclopentyl.

11. Compounds according to any of claims 1, 7 or 10, characterised in that the radical R denotes a straight-chain or methyl-branched alkyl or alkenyl radical with 5 to 12 carbon atoms.

12. A liquid-crystalline mixture containing one or more compounds according to any of claims 1 to 11.

13. A liquid crystalline mixture according to claim 12, characterised in that the other components therein are one or more compounds having the formulae: 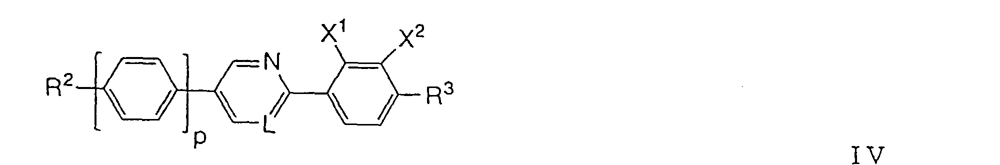 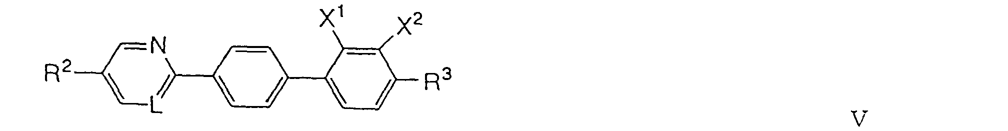 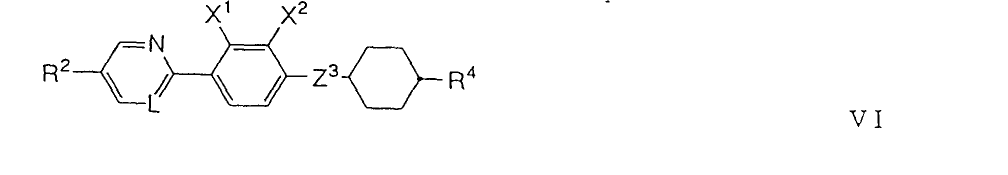 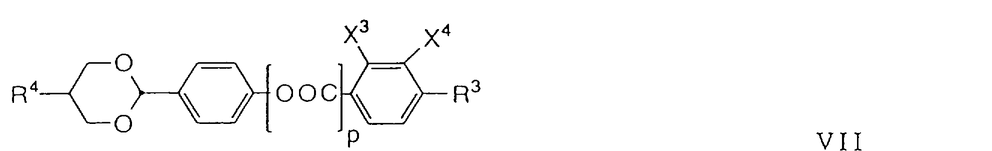 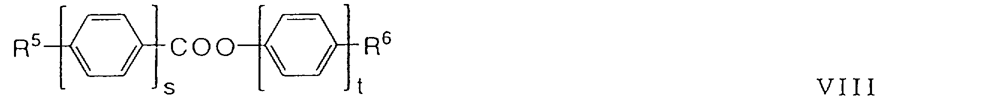 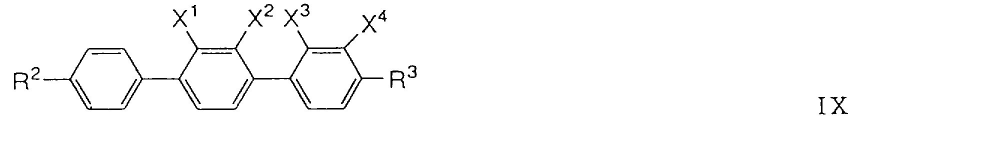 wherein
p is 0 or 1;
s, t denote 1 or 2, wherein s + t = 2 or 3;
L denotes nitrogen or -CH=;
X¹,X²,X³,X⁴ independently denote hydrogen or fluorine;
Z³ denotes a single bond, -OOC-, -OCH₂- or -(CH₂)₂-;
R²,R³ independently denote alkyl, alkenyl, alkoxy, alkoxyalkyl, alkenyloxy, alkanoyloxy, alkenoyloxy, alkoxyalkoxy, alkoxycarbonyl or alkenyloxy-carbonyl;
R⁴ denotes alkyl or alkenyl; and
R⁵, R⁶ independently denote alkyl, alkenyl, alkoxy or alkenyloxy.

14. A liquid-crystalline mixture according to claim 12 or 13, characterised in that it contains one or more optically active compounds from the group of compounds having the general formulae: 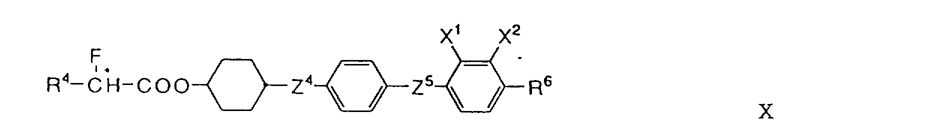 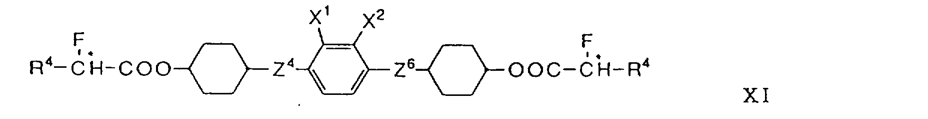 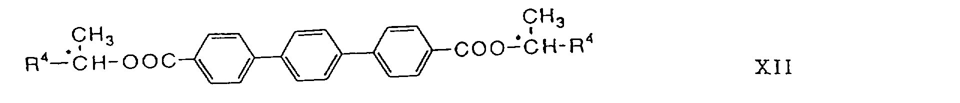 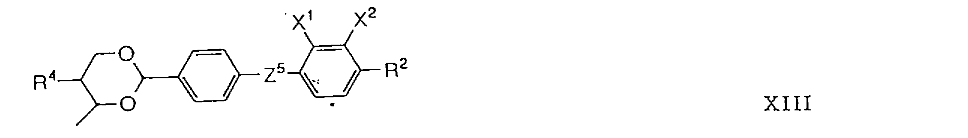 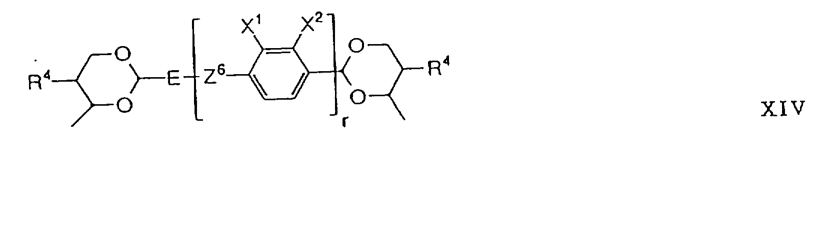 wherein
E denotes 1,4-phenylene or trans-1,4-cyclohexylene;
r is 0, 1 or 2;
Z⁴ denotes a single bond, -(CH₂)₂- or -CH₂O-;
Z⁵ denotes a single bond, -OCH₂-, -COO- or -OOC-;
Z⁶ denotes a single bond, -(CH₂)₂- or -OCH₂-
and the other symbols have the meanings given in Claim 12.

15. Use of liquid-crystalline compounds according to any of claims 1 to 10 for optical and electro-optical devices.

16. Use of liquid-crystalline mixtures according to any of claims 11 to 14 for optical and electro-optical devices.

17. Optical and electro-optical devices according to any of claims 1 to 15 and based on the DHF-, SBF or SSF- effect.

## Revendications

1. Composés du cyclopentyle ayant la formule générale I 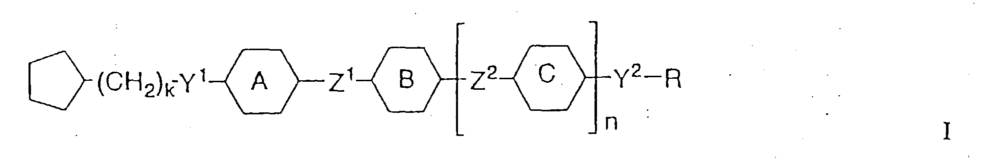 où
k représente un nombre entier de 4 à 18 ;
n est 0 ou 1 ;
Y¹, Y² représentent une liaison simple, -O-, -COO-, ou -OOC- ;
les cycles A, B, C, indépendamment les uns des autres, représentent le 1,4-phénylène, le cas échéant mono- ou difluoré, la pyrimidine-2,5-diyle, la pyridine-2,5-diyle, la pyrazine-2,5-diyle, le naphtalène-2,6-diyle, la quinoléine-2,6-diyle, le thiophène-2,5-diyle, le thiazole-2,5-diyle, ou le 1,3,4-thiadiazole-2,5-diyle, et le cycle C représente aussi le trans-1,4-cyclohexylène ou le trans-1,3-dioxanne-2,5-diyle ;
Z¹ représente une liaison simple, -COO-, -OOC- ou -C≡C- ;
Z² représente une liaison simple, -COO-, -OOC-, -OCH₂-, -CH₂O- ou -(CH₂)₂- ; et
R représente un alkyle ou un alcényle, à chaîne droite ou ramifié, le cas échéant optiquement actif, ayant de 4 à 20 atomes de carbone, où un ou deux groupes méthylènes non voisins peuvent être remplacés par -O-, -COO-, -OOC- et/ou l'époxyéthylène et/ou au moins un hydrogène peut être remplacé par le fluor et/ou un hydrogène peut être remplacé par le chlore ou le cyano et/ou un hydrogène terminal peut être remplacé par le cyclopentyle.

2. Composés selon la revendication 1, caractérisés en ce que les cycles A, B et C, indépendamment les uns des autres, représentent le 1,4-phénylène, le 2- ou 3-fluoro-1,4-phénylène, le 2,3-difluoro-1,4-phénylène, la pyrimidine-2,5-diyle, la pyridine-2,5-diyle, ou la pyrazine-2,5-diyle, et le cycle C représente aussi le trans-1,4-cyclohexylène ou le trans-1,3-dioxanne-2,5-diyle.

3. Composés selon une des revendications 1 ou 2, caractérisés en ce que au moins un des cycles A, B ou C représente le 1,4-phénylène, le 2- ou 3-fluoro-1,4-phénylène, ou le 2,3-difluoro-1,4-phénylène, et au plus un des cycles A, B ou C représente la pyrimidine-2,5-diyle, la pyridine-2,5-diyle, ou la pyrazine-2,5-diyle.

4. Composés des formules Ia à Ii : 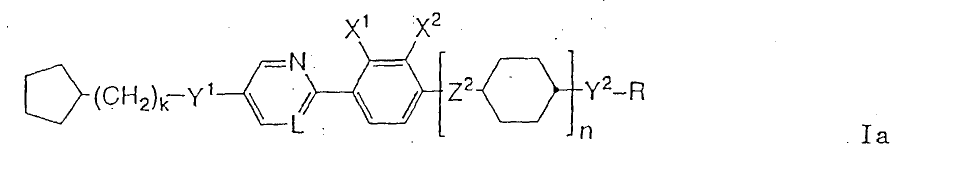 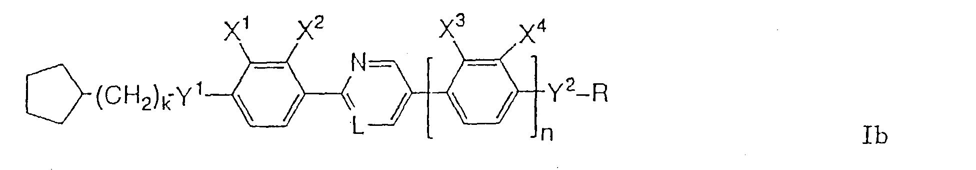 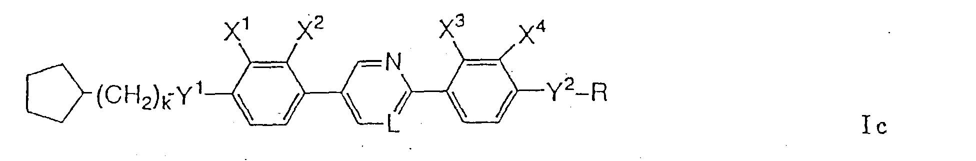 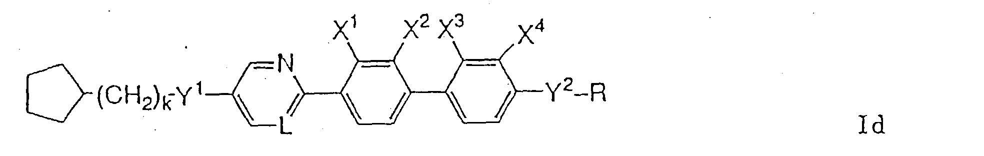 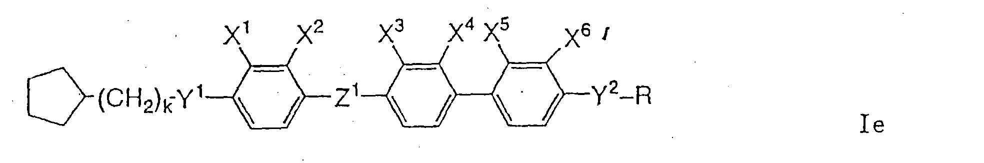 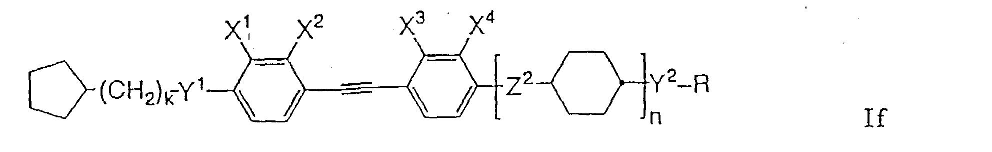 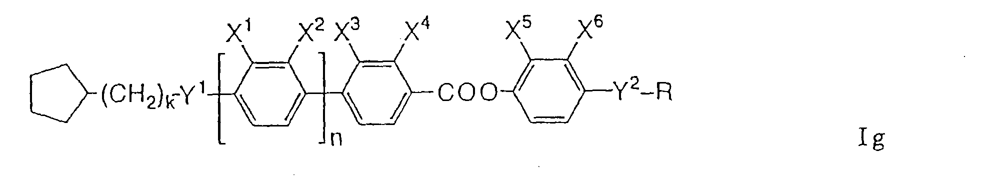 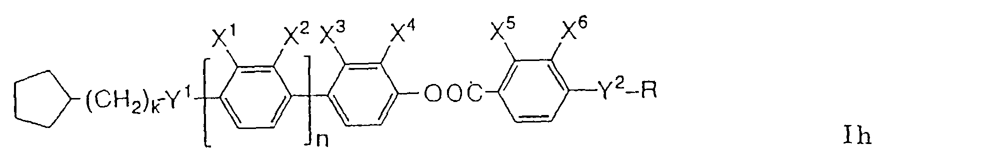 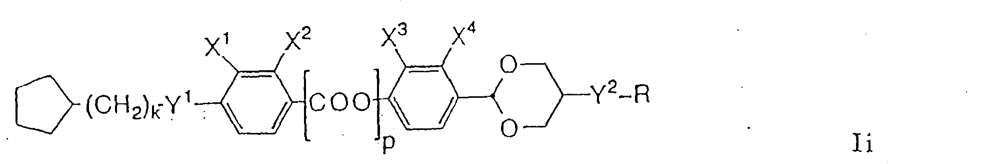 où
L représente l'azote ou -CH=;
X¹⁻⁶ représentent l'hydrogène ou le fluor ;
p est 0 ou 1 ; et
n, k, Y¹, Y² et R ont les significations données dans la revendication 1.

5. Composés selon une des revendications 1 à 4, caractérisés en ce que k est un nombre entier de 5 à 12.

6. Composés selon une des revendications 1 à 5, caractérisés en ce que Y¹ représente une liaison simple, -O- ou -COO-.

7. Composés selon une des revendications 1 à 6, caractérisés en ce que Y¹ représente -O-.

8. Composés selon une des revendications 1 à 7, caractérisés en ce que le reste R est un reste alkyle ou alcényle ayant de 5 à 12 atomes de carbone, où de 1 à 2 groupes méthylènes non terminaux sont remplacés par -C*H(W)-, -C*F(CH₃)- et/ou l'époxyéthylène, et où 1 ou 2 groupes méthylènes non voisins peuvent être remplacés par -O-, -COO-, -OOC- ; W représente le fluor, le chlore, le cyano, le méthyle ou le trifluorométhyle, et C* représente un centre chiral.

9. Composés selon une des revendications 1 à 8, caractérisés en ce que le groupe -Y²- R représente un groupe optiquement actif comme un 2- ou 3-fluoroalkyle, un 2- ou 3-fluoroalcoxy, un 2,3-difluoroalcoxy, un 2- ou 3-fluoro-alcanoyloxy, un 2,3-difluoroalcanoyloxy, un 2-fluoro-2-méthyl-alcanoyloxy, un 2-fluoro-3-méthyl-alcanoyloxy, un 2- ou 3-chloro-alcoxy, un 2- ou 3-chloro-alcanoyloxy, un 2-chloro-3-méthylalcanoyloxy, un 1- ou 2-cyanalkyle, un 1- ou 2-cyanalcoxy, un 2- ou 3- cyanalcanoyloxy, un 1-, 2- ou 3-méthylalkyle, un 1-, 2- ou 3-méthylalcoxy, un 2- ou 3-méthyl-alcanoyloxy, un 1-, 2- ou 3-trifluorométhyl-alcanoyloxy, un 1,2-époxyalkyle, un 2,3-époxyalcoxy, un 2,3-époxy-alcanoyloxy, un 1-alcoxycarbonyl-éthyle, un 1-alcoxycarbonyl-éthoxy, un 2-alcoxy-propanoyloxy, un (1-méthyl-alcoxy)-carbonyle, un (1-trifluorométhyl-alcoxy)-carbonyle, un 1-alcoxy-2,2,2-trifluoroéthyle, un ω-trifluoro-méthyl-ω-alcoxyalkyle, avec chaque fois de 5 à 12 atomes de carbone.

10. Composés selon une des revendications 1 à 7, caractérisés en ce que Y² est une liaison simple, -O- ou -OOC- ; et le reste R est un reste alkyle ou alcényle à chaîne droite ou ramifié (racémique) ayant de 5 à 12 atonies de carbone, où un groupe méthylène non voisin de Y² peut être remplacé par -O-, -COO- ou -OOC- et/ou un ou plusieurs atomes d'hydrogène peuvent être remplacés par le fluor et/ou un atome d'hydrogène terminal peut être remplacé par le cyclopentyle.

11. Composés selon une des revendications 1 à 7 ou 10, caractérisés en ce que le reste R est un reste alkyle ou alcényle à chaîne droite ou méthyl-ramifié ayant de 5 à 12 atomes de carbone.

12. Mélange à cristaux liquides comportant un ou plusieurs composés selon une des revendications 1 à 11.

13. Mélange à cristaux liquides selon la revendication 12, caractérisé en ce qu'il comporte comme autres composants un ou plusieurs composés des formules IV à IX 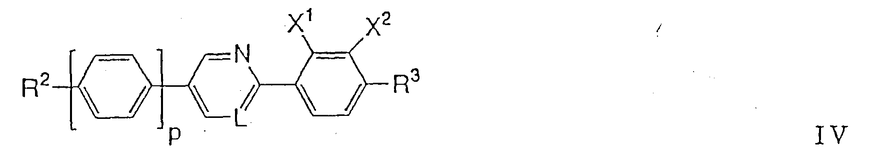 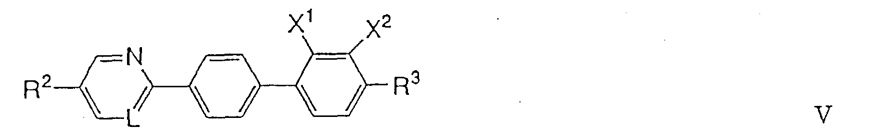 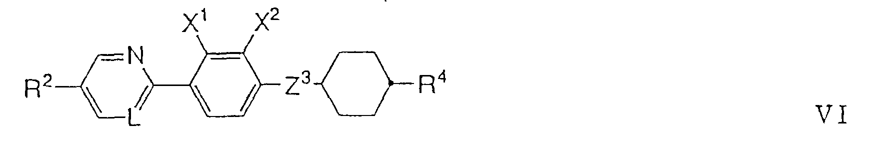 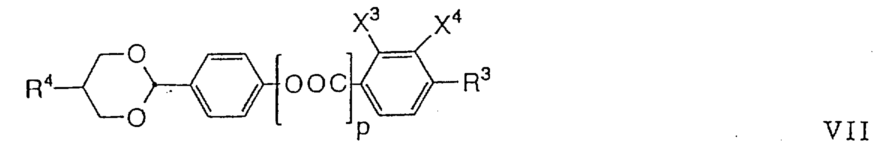 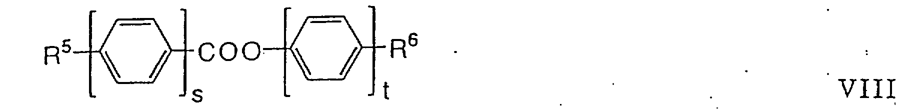 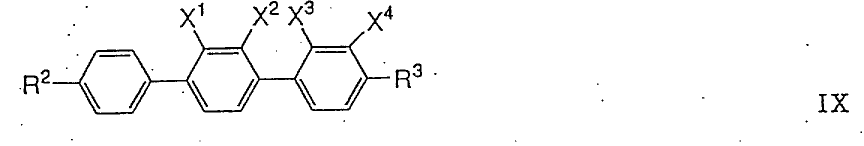 où
p est 0 ou 1;
s,t sont 1 ou 2, où s + t = 2 ou 3 ;
L est l'azote ou -CH= ;
X¹, X², X³, X⁴, indépendamment les uns des autres, représentent l'hydrogène ou le fluor ;
Z³ représente une liaison simple, -OOC-, -OCH₂-, ou -(CH₂)₂- ;
R², R³, indépendamment l'un de l'autre, représentent un alkyle, un alcényle, un alcoxy, un alcoxyalkyle, un alcényloxy, un alcanoyloxy, un alcénoyloxy, un alcoxyalcoxy, un alcoxycarbonyle ou un alcényloxycarbonyle ;
R⁴ représente un alkyle ou un alcényle ;
R⁵, R⁶, indépendamment l'un de l'autre, représentent un alkyle, un alcényle, un alcoxy ou un alcényloxy.

14. Mélange à cristaux liquides selon une des revendications 12 ou 13, caractérisé en ce qu'il comporte un ou plusieurs composés optiquement actifs à partir du groupe des composés des formules générales X à XIV 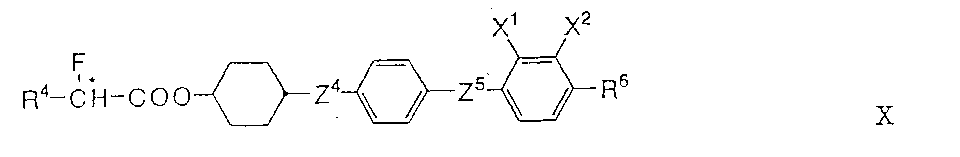 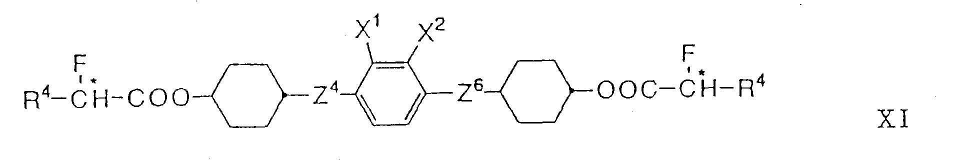 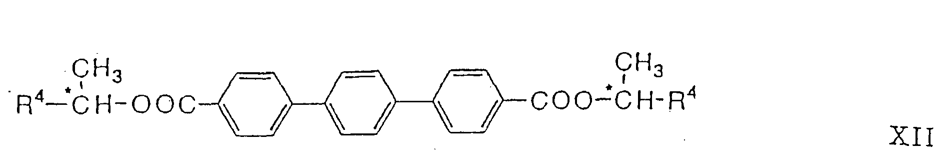 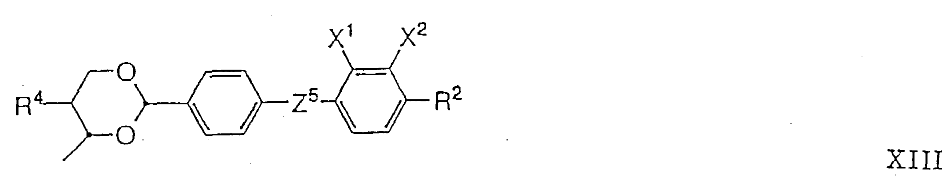 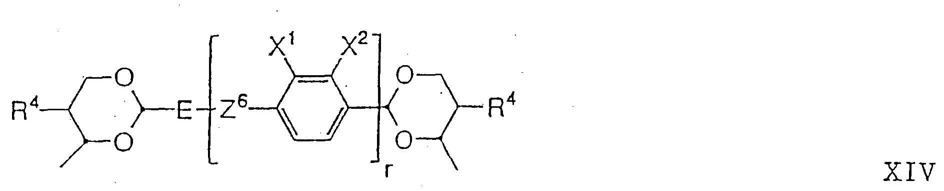 où
E représente le 1,4-phénylène ou le trans 1,4-cyclohexylène ;
r est 0, 1 ou 2 ;
Z⁴ représente une liaison simple, -(CH₂)₂- ou -CH₂O- ;
Z⁵ représente une liaison simple, -OCH₂-, -COO- ou -OOC- ;
Z⁶ représente une liaison simple, -(CH₂)₂- ou -OCH₂- ;
et les autres symboles ont les significations données dans la revendication 12.

15. Utilisation de composés cristaux liquides selon une des revendications 1 à 10 pour des dispositifs optiques et électro-optiques.

16. Utilisation de mélanges à cristaux liquides selon une des revendications 11 à 14 pour des dispositifs optiques et électro-optiques.

17. Dispositifs optiques et électro-optiques selon une des revendications 1 et 15 à base des effets DHF, SBF ou SSF.
